# EUROPEAN PATENT SPECIFICATION

(11) **EP 4 623 403 B1**
(45) Date of publication and mention of the grant of the patent: **02.12.2026**
(21) Application number: 22969861.8
(22) Date of filing: 30.12.2022
(51) Int. Cl.: G06T 5/00, G06T 11/00, G06N 3/02, A61B 5/055, G01R 33/56, A61B 5/00, G06N 3/045, G06T 3/4046, G06T 5/50, G06T 5/60, G06T 5/70, G06N 3/08, G06N 3/084, G06N 3/09

(54) **SYSTEMS AND METHODS FOR IMAGE OPTIMIZATION**
SYSTEME UND VERFAHREN ZUR BILDOPTIMIERUNG
SYSTÈMES ET PROCÉDÉS D'OPTIMISATION D'IMAGES

(43) Date of publication of application: 01.10.2025
(73) Proprietor: Shanghai United Imaging Healthcare Co., Ltd., Shanghai 201807 (CN)
(72) Inventor: HUANG, Yanqi, Shanghai 201807 (CN); HUANG, Yukun, Shanghai 201807 (CN)
(74) Representative: Wang, Bo
(86) International application number: PCT/CN2022/144263
(87) International publication number: WO 2024/138719

(56) References cited:
- CN-A- 109 697 740
- CN-A- 111 932 461
- CN-A- 112 017 113
- CN-A- 113 284 046
- CN-A- 114 252 423
- CN-A- 114 487 964
- US-A1- 2020 072 931
- LI GUANGYUAN ET AL: "Transformer-empowered Multi-scale Contextual Matching and Aggregation for Multi-contrast MRI Super-resolution", 2022 IEEE/CVF CONFERENCE ON COMPUTER VISION AND PATTERN RECOGNITION (CVPR), IEEE, 18 June 2022 (2022-06-18), pages 20604 - 20613, XP034193529, DOI: 10.1109/CVPR52688.2022.01998
- TSANG COLIN S C ET AL: "Joint Denoising and Super-Resolution for Fluorescence Microscopy Using Weakly-Supervised Deep Learning", 15 September 2022, SPRINGER INTERNATIONAL PUBLISHING, PAGE(S) 32 - 41, XP047633963
- MA CHENG ET AL: "Structure-Preserving Super Resolution With Gradient Guidance", 2020 IEEE/CVF CONFERENCE ON COMPUTER VISION AND PATTERN RECOGNITION (CVPR) IEEE, 13 June 2020 (2020-06-13), pages 7766 - 7775, XP033805259, [retrieved on 20200803], DOI: 10.1109/CVPR42600.2020.00779

## Description

### TECHNICAL FIELD

The disclosure generally relates to image processing, and more particularly relates to systems and methods for image optimization.

### BACKGROUND

Magnetic resonance imaging (MRI) is a significant imaging technology and is widely used in disease diagnosis and/or treatment of various medical conditions. Magnetic resonance (MR) images, which are acquired using an MRI device, may have an image quality unsatisfactory for a clinical need, which may be due to, e.g., the resolution of the MRI device and/or acquisition modes of the MR images. For example, the MR images may be with low resolution and/or low signal-to-noise ratio (SNR). LI GUANGYUAN ET AL: "Transformer-empowered Multi-scale Contextual Matching and Aggregation for Multi-contrast MRI Super-resolution", 2022, disclose a super-resolution machine learning model for MRI images. TSANG COLIN S C ET AL: "Joint Denoising and Super-Resolution for Fluorescence Microscopy Using Weakly-Supervised Deep Learning" discloses a model for joint denoising and super-resolution of microscopy image. Therefore, it is desirable to provide systems and methods for image optimization, thereby improving the image quality of the MR images.

### SUMMARY

The present invention is defined by the appended claims. In one aspect of the present disclosure, a method for image optimization is provided. The method may include obtaining an initial image of a target object. The initial image may have a first image quality. The method may also include obtaining a correlation reference image that is generated based on a reference image associated with the target object. The reference image may have a second image quality higher than the first image quality and the correlation reference image may have a third image quality lower than the second image quality. The method may further include determining an optimized image of the initial image by inputting the initial image and the correlation reference image to an optimization model. The optimization model may refer to a machine learning model that is configured for high-resolved and noise-reduced reconstruction using priori information existing in the refence image. The optimized image may have a fourth image quality higher than the first image quality.

In another aspect of the present disclosure, a system for image optimization is provided. The systems may include a storage device including a set of instructions and at least one processor in communication with the storage device. When executing the set of instructions, the at least one processor the at least one processor may be configured to direct the system to perform following operations. The system may obtain an initial image of a target object. The systems may also obtain a correlation reference image that is generated based on a reference image associated with the target object. The systems may further determine an optimized image of the initial image by inputting the initial image and the correlation reference image to an optimization model as described above.

In another aspect of the present disclosure, a system for image optimization is provided. The system may include an obtaining module configured to obtain an initial image of a target object and obtain a correlation reference image that is generated based on a reference image associated with the target object. The initial image may have a first image quality. The system may also include a determination module configured to determine an optimized image of the initial image by inputting the initial image and the correlation reference image to an optimization model as described above.

In another aspect of the present disclosure, a non-transitory computer readable medium is provided. The non-transitory computer readable medium may include executable instructions that, when executed by at least one processor, direct the at least one processor to perform a method for image optimization as described above.

In another aspect of eth present disclosure, a method for generating an optimization model is provided. The method may be implemented by a computing device. The method may include obtaining a plurality of training samples each of which includes a sample image of a sample object, a sample reference image associated with the sample object, and a sample gold standard image corresponding to the sample image. The sample image may have a first image quality, the sample reference image may have a second image quality higher than the first image quality, and the sample gold standard image may have a third image quality higher than the first image quality. The method may also include obtaining an initial machine learning model. The method may further include generating the optimization model by training, using the plurality of training samples, the initial machine learning model according to a training process. The training process may include for each of the plurality of training samples, determining a sample correlation reference image based on the sample reference image, the sample correction reference image having a fourth image quality lower than the second image quality; and generating the optimization model using each of the plurality of training samples and a corresponding sample correlation reference image.

In another aspect of the present disclosure, a system for generating an optimization model is provided. The system may include a storage device including a set of instructions and at least one processor in communication of the storage device. When executing the set of instructions, the at least one processor may be configured to direct the system to perform following operations. The system may obtain a plurality of training samples. The system may also obtain an initial machine learning model. The system may further generate the optimization model by training, using the plurality of training samples, the initial machine learning model according to a training process as described above. The training process may include for each of the plurality of training samples, determining a sample correlation reference image based on the sample reference image. The sample correction reference image may have a fourth image quality lower than the second image quality. The training process may also include generating the optimization model using each of the plurality of training samples and a corresponding sample correlation reference image.

In another aspect, a system for generating an optimization model is provided. The system may include an obtaining module and a training module. The obtaining module may be configured to obtain a plurality of training samples. The obtaining module may also be configured to obtain an initial machine learning model. The training module may be configured to generate the optimization model by training, using the plurality of training samples, the initial machine learning model according to a training process as described above.

In another aspect of the present disclosure, a non-transitory computer readable medium is provided. The non-transitory computer readable medium may include executable instructions that, when executed by at least one processor, direct the at least one processor to perform a method for generating an optimization model as described above.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary imaging system according to some embodiments of the present disclosure;
FIG. 2 is a schematic diagram illustrating hardware and/or software components of an exemplary computing device according to some embodiments of the present disclosure;
FIG. 3 is a schematic diagram illustrating hardware and/or software components of an exemplary mobile device according to some embodiments of the present disclosure;
FIG. 4A and FIG. 4B are block diagrams illustrating exemplary processing devices according to some embodiments of the present disclosure;
FIG. 5 is a flowchart illustrating an exemplary process for image optimization according to some embodiments of the present disclosure;
FIG. 6 is a schematic diagram illustrating an application of an exemplary optimization model according to some embodiments of the present disclosure;
FIG. 7 is a flowchart illustrating an exemplary process for generating an optimization model according to some embodiments of the present disclosure; and
FIG. 8 is a flowchart illustrating an exemplary training process according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure. Various modifications to the disclosed embodiments will be readily apparent to those skilled in the art, and the general principles defined herein may be applied to other embodiments and applications. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof. The term "image" in the present disclosure is used to collectively refer to image data (e.g., scan data) and/or images of various forms, including a two-dimensional (2D) image, a three-dimensional (3D) image, a four-dimensional (4D) image, etc. The term "pixel" and "voxel" in the present disclosure are used interchangeably to refer to an element of an image.

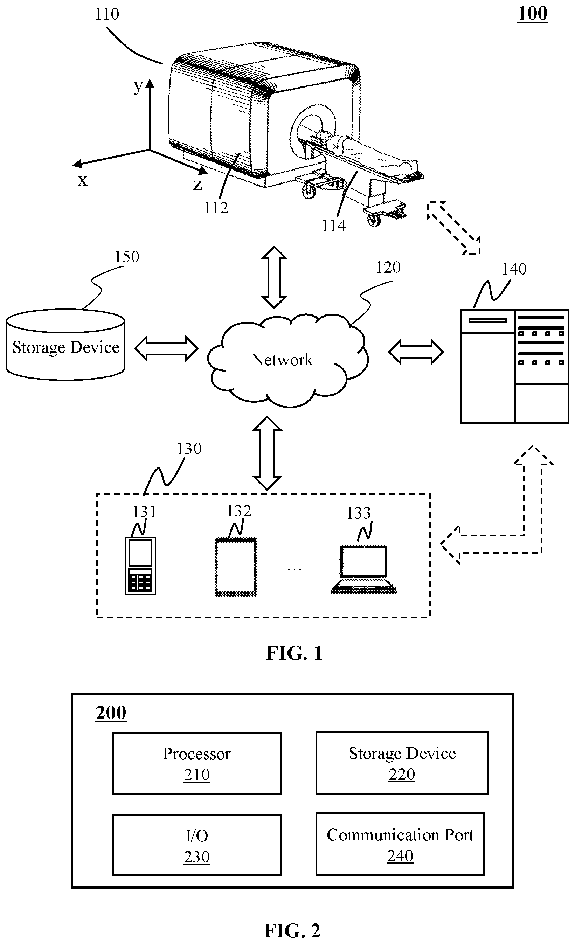

It will be understood that the term "system," "engine," "unit," "module," and/or "block" used herein are one method to distinguish different components, elements, parts, sections or assembly of different levels in ascending order. However, the terms may be displaced by another expression if they achieve the same purpose. Generally, the word "module," "unit," or "block," as used herein, refers to logic embodied in hardware or firmware, or to a collection of software instructions. A module, a unit, or a block described herein may be implemented as software and/or hardware and may be stored in any type of non-transitory computer-readable medium or another storage device. In some embodiments, a software module/unit/block may be compiled and linked into an executable program. It will be appreciated that software modules can be callable from other modules/units/blocks or from themselves, and/or may be invoked in response to detected events or interrupts. Software modules/units/blocks configured for execution on computing devices (e.g., processor 210 as illustrated in FIG. 2) may be provided on a computer-readable medium, such as a compact disc, a digital video disc, a flash drive, a magnetic disc, or any other tangible medium, or as a digital download (and can be originally stored in a compressed or installable format that needs installation, decompression, or decryption prior to execution). Such software code may be stored, partially or fully, on a storage device of the executing computing device, for execution by the computing device. Software instructions may be embedded in firmware, such as an EPROM. It will be further appreciated that hardware modules/units/blocks may be included in connected logic components, such as gates and flip-flops, and/or can be included of programmable units, such as programmable gate arrays or processors. The modules/units/blocks or computing device functionality described herein may be implemented as software modules/units/blocks, but may be represented in hardware or firmware. In general, the modules/units/blocks described herein refer to logical modules/units/blocks that may be combined with other modules/units/blocks or divided into sub-modules/sub-units/sub-blocks despite their physical organization or storage. The description may be applicable to a system, an engine, or a portion thereof.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

Provided herein are systems and methods for non-invasive biomedical imaging, such as for disease diagnostic or research purposes. In some embodiments, the systems may include a single modality imaging system and/or a multi-modality imaging system. The single modality imaging system may include, for example, a PET system and a computed tomography (CT) system. The multi-modality imaging system may include, for example, a positron emission tomography-computed tomography (PET-CT) system, etc. It should be noted that the imaging system described below is merely provided for illustration purposes, and not intended to limit the scope of the present disclosure.

The term "imaging modality" or "modality" as used herein broadly refers to an imaging method or technology that gathers, generates, processes, and/or analyzes imaging information of an object. The object may include a biological object and/or a non-biological object. The biological object may be a human being, an animal, a plant, or a portion thereof (e.g., a cell, a tissue, an organ, etc.). In some embodiments, the object may be a man-made composition of organic and/or inorganic matters that are with or without life. The term "object" or "subject" are used interchangeably.

In the present disclosure, the term "image" may refer to a two-dimensional (2D) image, a three-dimensional (3D) image, or a four-dimensional (4D) image. As used herein, a representation of a subject (e.g., a patient, or a portion thereof) in an image may be referred to as the subject for brevity. For instance, a representation of an organ or tissue (e.g., the heart, the liver, a lung, etc., of a patient) in an image may be referred to as the organ or tissue for brevity. An image including a representation of a subject may be referred to as an image of the subject or an image including the subject for brevity. As used herein, an operation on a representation of a subject in an image may be referred to as an operation on the subject for brevity. For instance, a segmentation of a portion of an image including a representation of an organ or tissue (e.g., the heart, the liver, a lung, etc., of a patient) from the image may be referred to as a segmentation of the organ or tissue for brevity.

In some embodiments, a subject may be imaged using an MRI device to obtain an MR image of the subject in multiple situations. The MRI device may include a volume transmitting coil (VTC) and/or one or more surface coils. For example, in a radiation therapy planning, the surface coil(s) of the MRI device may be used for imaging. When put on the subject, the surface coil(s) may press the subject, causing a change in the morphology and/or position of, e.g., muscle and/or an organ. Such a change may be reflected in an MR image so acquired, which in turn may affect the accuracy of the radiation therapy planned on the basis of the MR image. As another example, in a situation where the subject is with severe trauma (e.g., fractures) or is too weak, the usage of the surface coil(s) may increase the pain of the subject. Thus, the subject may need to be imaged without the surface coil(s) but directly using the VTC of the MRI device. The MR image of the subject may be generated based on MR signals received by the VTC of the MRI device. An MR imaging scan using the VTC may obviate the need for an operator (e.g., a medical staffer such as a doctor, or a technician) to set up surface coil(s), thereby improving the efficiency of the imaging and/or radiotherapy planning (e.g., in a multi-modality scan such as an MRI/PET scan). However, as the VTC covers a larger region (e.g., tissue) than the surface coil(s) and the VTC is further away from the region (e.g., the tissue) to be imaged than the surface coil(s), under a same spatial resolution, the SNR of signals acquired using the VTC may be lower than the SNR of signals acquired using the surface coil(s), and the MR image of the subject generated based on signals acquired using the VTC may have a relatively low image quality than an MR image of the subject generated based on signals acquired using the surface coil(s). Alternatively, the subject may be imaged by an MRI device with a low spatial resolution (e.g., a low-field MRI device), e.g., subject to the medical condition. Thus, an image of the subject acquired by the MRI device with a low spatial resolution may have a lower image quality and/or lower SNR than an image of the subject acquired by an MRI device with a high spatial resolution (e.g., a high-field MRI device). Therefore, it is desirable to improve the resolution and/or SNR of an MR image that is acquired using a VTC and/or a low-field MRI device.

An aspect of the present disclosure relates to systems and methods for image optimization. The systems may obtain an initial image (e.g., an initial MR image) of a target object. The initial image may have a first image quality. The systems may obtain a reference image (e.g., a reference MR image) associated with the target object. The reference image may have a second image quality higher than the first image quality. The systems may generate a correlation reference image based on the reference image. The correlation reference image may have a third image quality lower than the second image quality (e.g., consistent with the first image quality). The systems may determine an optimized image of the initial image by inputting the initial image, the reference image, and the correlation reference image to an optimization model. The optimization model may include a deep feature extraction component and a correlation search component. The optimized image may have a fourth image quality higher than the first image quality (e.g., consistent with the second image quality).

According to some embodiments of the present disclosure, the image (e.g., an MRI image) with low image quality may be accurately processed to obtain an image with improved image quality (e.g., the image with improved image quality being with higher-resolution and/or lower noise than that of the image with low image quality). For example, an image with high image quality may be taken as a reference image, and deep features of the reference image may be extracted as priori information. Structural features of the image with low image quality may be extracted by a structural feature extraction component (e.g., a structural extraction network with multi-channel and multi-module) which can also remove noises that may obscure the structural features. By using a correlation search component (e.g., a correlation search network), a mapping relationship between features of the image with high image quality and features of the image with low image quality can be determined. According to the mapping relationship, the features of the image with high image quality may be transferred to the image with low image quality for restoring fine structures corresponding to the subject in the image with low image quality, thereby achieving high resolution and/or low noise of the image with improved image quality. In such cases, imaging using the VTC of the MRI device can be employed for an image-guided radiation therapy, which can avoid the interference of the surface coil(s) and the holder thereof with radiation therapy positioning, make full use of the bore space of the MRI device, and allow multiple desired positionings of the radiation therapy and simulation of the physical status of the subject during radiation therapy. In addition, imaging using the VTC, instead of the surface coil(s), can simplify the setup operation, and improve the stability of imaging quality of the MRI device. Furthermore, the MRI device with low spatial resolution can be directly used to scan the target object and obtain the improved image by using a reference image with high image quality and an optimization model, which can reduce costs and improve the examination efficiency, in composition with using an MRI device with high spatial resolution for a scan.

Another aspect of the present disclosure relates to systems and methods for generating an optimization model. The systems may obtain a plurality of training samples each of which includes a sample image of a sample object, a sample reference image associated with the sample object, and a sample gold standard image corresponding to the sample image. The sample image may have a first image quality. The sample reference image may have a second image quality higher than the first image quality. The sample gold standard image may have a third image quality higher than the first image quality. For example, the second image quality and the third image quality may be consistent with each other (e.g., equal to or a difference between thereof being less than a threshold). The systems may obtain an initial machine learning model. The systems may generate the optimization model by training, using the plurality of training samples, the initial machine learning model according to a training process. The training process may include for each of the plurality of training samples, determining a sample correlation reference image based on the sample reference image. The sample correction reference image may have a fourth image quality lower than the second image quality. The training process may also include generating the optimization model using each of the plurality of training samples and a corresponding sample correlation reference image.

According to some embodiments of the present disclosure, the optimization model may be trained using the sample image of the sample object, the sample reference image associated with the sample object, and the sample gold standard image corresponding to the sample image of each training sample. For a certain training sample, the corresponding reference image and the corresponding gold standard image may be images of the sample object. The optimization model may be applied in a situation where there is an existing reference image of the target object, and an optimized image of the target object is to be generated based on the optimization model, the reference image, and an image with low quality of the target object. Alternatively or additionally, the optimization model trained may be applied in a situation where there is no existing reference image of the target object, and an improved image of the target object is to be generated based on the optimization model, an image with low quality of the target object, and a reference image of another object.

FIG. 1 is a schematic diagram illustrating an exemplary imaging system according to some embodiments of the present disclosure. The imaging system 100 may include a single-modality system (e.g., an MRI system) or a multi-modality system (e.g., an MRI-guided radiotherapy device). For illustration purposes, the imaging system 100 illustrated in FIG. 1 may include an MRI system. The MRI system may include an imaging device such as an MRI scanner (also referred to as an MRI device) 110, a network 120, a terminal device 130, a processing device 140, and a storage device 150. The components of the imaging system 100 may be operably connected in one or more of various ways. Merely by way of example, as illustrated in FIG. 1, the imaging device 110 may be connected to the processing device 140 through the network 120. As another example, the imaging device 110 may be operably connected to the processing device 140 directly. As a further example, the storage device 150 may be operably connected to the processing device 140 directly or through the network 120. As still a further example, a terminal device (e.g., 131, 132, 133, etc.) may be operably connected to the processing device 140 directly or through the network 120. It should be noted that the imaging system 100 may include any other imaging device other than the MRI device, which is not limited herein.

In the present disclosure, the x-axis, the y-axis, and the z-axis shown in FIG. 1 may form an orthogonal coordinate system. The x-axis and the z-axis shown in FIG. 1 may be horizontal, and the y-axis may be vertical. As illustrated, the positive x-direction along the x-axis may be from the right side to the left side of the imaging device 110 seen from the direction facing the front of the imaging device 110; the positive y-direction along the y axis shown in FIG. 1 may be from the lower part to the upper part of the imaging device 110; the positive z-direction along the z-axis shown in FIG. 1 may refer to a direction in which the object is moved out of the detection region (or referred to as the bore) of the imaging device 110.

The imaging device 110 may be configured to scan at least a part of a subject and acquire image data (or scan data) relating to the subject. The imaging device (e.g., an MRI device) 110 may include magnets (not shown), coils (not shown), a gantry 112, a patient support 114, etc. The magnets of the imaging device may include a main magnet (e.g., a resistive magnet, a superconductive magnet, or a permanent magnet). The main magnet may form a bore (e.g., including a detection region) with an axis parallel to the z-direction as illustrated in FIG. 1 and surround the object that is moved into or positioned along the z-direction within the detection region. The main magnet may also control the homogeneity of the generated main magnetic field. The coils may include gradient coils, radio frequency (RF) coils, etc. The gradient coils may be located inside the main magnet (e.g., located in the bore formed by the main magnet). The gradient coils may be surrounded by the main magnet around the z-direction, and be closer to the object than the main magnet. The gradient coils may be configured to generate a gradient magnetic field. The gradient magnetic field may be superimposed on the main magnetic field generated by the main magnet and distort the main magnetic field so that the magnetic orientations of the protons of an object may vary as a function of their positions inside the gradient magnetic field, thereby encoding spatial information into MR signals generated by the region of the object being imaged. The RF coils may be located in the bore formed by the main magnet and serve as transmitters, receivers, or both.

When used as transmitters, the RF coils may generate RF signals that provide a magnetic field that is utilized to generate MR signals related to the region of the object being imaged. When used as receivers, the RF coils may be responsible for detecting MR signals (e.g., echoes). For example, the RF coils may include volume transmitting coils (VTCs), surface coils, or the like, or any combination thereof, for detecting MR signals. The surface coils may be located closer to the region being imaged than the VTCs. The surface coils may include different specialized coils (e.g., head coil(s), spin coil(s), body surface coil(s), neck coil(s), limb coil(s), etc.) for different parts of the object, which can improve signal conversation efficiency and/or image quality of an image determined based on MR signals so acquired. However, the surface coils may need to be set next to the surface of the object and be fixed by auxiliary facilities (e.g., a bandage, a holder, etc.), which limits the usage of the surface coils. The gantry 112 may be configured to support the magnets (e.g., the main magnet), the coils (e.g., the gradient coils and/or the RF coils), etc. The gantry 112 may surround, along the z-direction, the object that is moved into or located within the detection region. The patient support 114 may be configured to support the object. Accordingly, the position of the object within the detection region may be adjusted by adjusting the patient support 114. Merely by way of example, the patient support 114 may move the object into the detection region along the z-direction in FIG. 1.

The network 120 may include any suitable network that can facilitate the exchange of information and/or data for the imaging system 100. In some embodiments, one or more components of the imaging system 100 (e.g., the imaging device 110, the terminal device 130, the processing device 140, or the storage device 150) may communicate information and/or data with one or more other components of the imaging system 100 via the network 120. In some embodiments, the network 120 may be any type of wired or wireless network, or a combination thereof.

The terminal device 130 may include a mobile device 131, a tablet computer 132, a laptop computer 133, or the like, or any combination thereof. In some embodiments, the mobile device 131 may include a smart home device, a wearable device, a smart mobile device, a virtual reality device, an augmented reality device, or the like, or any combination thereof. In some embodiments, the terminal device 130 may remotely operate the imaging device 110 and/or the processing device 140. In some embodiments, the terminal device 130 may operate the imaging device 110 and/or the processing device 140 via a wireless connection. In some embodiments, the terminal device 130 may receive information and/or instructions inputted by a user, and send the received information and/or instructions to the imaging device 110 or to the processing device 140 via the network 120. In some embodiments, the terminal device 130 may receive data and/or information from the processing device 140. In some embodiments, the terminal device 130 may be part of the processing device 140. In some embodiments, the terminal device 130 may be omitted.

The processing device 140 may process data and/or information obtained from the imaging device 110, the terminal device 130, and/or the storage device 150. For example, the processing device 140 may obtain an initial image of a target object. The initial image may have a first image quality. The processing device 140 may obtain a reference image associated with the target object. The reference image may have a second image quality higher than the first image quality. The processing device 140 may generate a correlation reference image based on the reference image. The processing device 140 may determine an optimized image of the initial image by inputting the initial image, the reference image, and the correlation reference image to an optimization model. The optimization model may include a deep feature extraction component and a correlation search component. The optimized image may have a fourth image quality higher than the first image quality. As another example, the processing device 140 may generate an optimization model by training, using a plurality of training samples, an initial machine learning model according to a training process.

In some embodiments, the generation (e.g., training) and/or updating of the optimization model may be performed on a processing device, while the application of the optimization model may be performed on a different processing device. In some embodiments, the generation and/or updating of the optimization model may be performed on a processing device of a system different from the imaging system 100 or a server different from a server including the processing device 140 on which the application of the optimization model is performed. For instance, the generation and/or updating of the optimization model may be performed on a first system of a vendor who provides and/or maintains such an optimization model and/or has access to training samples used to generate the optimization model, while image optimization based on the provided optimization model may be performed on a second system of a client of the vendor. In some embodiments, the generation and/or updating of the optimization model may be performed on a first processing device of the imaging system 100, while the application of the optimization model may be performed on a second processing device of the imaging system 100. In some embodiments, the generation and/or updating of the optimization model may be performed online in response to a request for image optimization. In some embodiments, the generation and/or updating of the optimization model may be performed offline.

In some embodiments, the optimization model may be generated (e.g., trained) and/or updated (or maintained) by, e.g., the manufacturer of the imaging device 110 or a vendor. For instance, the manufacturer or the vendor may load the optimization model into the imaging system 100 or a portion thereof (e.g., the processing device 140) before or during the installation of the imaging device 110 and/or the processing device 140, and maintain or update the optimization model from time to time (periodically or not). The maintenance or update may be achieved by installing a program stored on a storage device (e.g., a compact disc, a USB drive, etc.) or retrieved from an external source (e.g., a server maintained by the manufacturer or vendor) via the network 120. The program may include a new model (e.g., a new optimization model) or a portion thereof that substitutes or supplements a corresponding portion of the optimization model.

In some embodiments, the processing device 140 may be a single server, or a server group. The server group may be centralized or distributed. In some embodiments, the processing device 140 may be local or remote. In some embodiments, the processing device 140 may be implemented on a cloud platform. For example, a cloud platform may include a private cloud, a public cloud, a hybrid cloud, a community cloud, a distributed cloud, an inter-cloud, and a multi-cloud, or the like, or any combination thereof. In some embodiments, the processing device 140 may be implemented by a computing device 200 having one or more components as illustrated in FIG. 2.

The storage device 150 may store data and/or instructions. In some embodiments, the storage device 150 may store data obtained from the imaging device 110, the terminal device 130, and/or the processing device 140. In some embodiments, the storage device 150 may store data and/or instructions that the processing device 140 may execute or use to perform exemplary methods described in the present disclosure. In some embodiments, the storage device 150 may include a mass storage device, a removable storage device, a volatile read-and-write memory, a read-only memory (ROM), or the like, or any combination thereof. In some embodiments, the storage device 150 may be implemented on a cloud platform. For example, a cloud platform may include a private cloud, a public cloud, a hybrid cloud, a community cloud, a distributed cloud, an inter-cloud, a multi-cloud, or the like, or any combination thereof.

In some embodiments, the storage device 150 may be connected to the network 120 to communicate with one or more components of the imaging system 100 (e.g., the imaging device 110, the processing device 140, the terminal device 130, etc.). One or more components of the imaging system 100 may access the data or instructions stored in the storage device 150 via the network 120. In some embodiments, the storage device 150 may be part of the processing device 140 or may be independent and directly or indirectly connected to the processing device 140.

It should be noted that the above description of the imaging system 100 is merely provided for the purposes of illustration, and not intended to limit the scope of the present disclosure. For persons having ordinary skills in the art, multiple variations and modifications may be made under the teachings of the present disclosure. For example, the imaging system 100 may include one or more additional components and/or one or more components of the imaging system 100 described above may be omitted. Additionally or alternatively, two or more components of the imaging system 100 may be integrated into a single component. A component of the imaging system 100 may be implemented on two or more sub-components.

FIG. 2 is a schematic diagram illustrating hardware and/or software components of an exemplary computing device 200 may be implemented according to some embodiments of the present disclosure. The computing device 200 may be used to implement any component of the imaging system as described herein. For example, the processing device 140 and/or a terminal device 130 may be implemented on the computing device 200, respectively, via its hardware, software program, firmware, or a combination thereof. Although only one such computing device is shown, for convenience, the computer functions relating to the imaging system 100 as described herein may be implemented in a distributed fashion on a number of similar platforms, to distribute the processing load. As illustrated in FIG. 2, the computing device 200 may include a processor 210, a storage device 220, an input/output (I/O) 230, and a communication port 240.

The processor 210 may execute computer instructions (program codes) and perform functions of the processing device 140 in accordance with techniques described herein. The computer instructions may include, for example, routines, programs, objects, components, signals, data structures, procedures, modules, and functions, which perform particular functions described herein. For example, the processor 210 may perform attenuation correction on a PET image to generate an optimized image of a target object. As another example, the processor 210 may generate an optimization model according to a machine learning technique. In some embodiments, the processor 210 may perform instructions obtained from the terminal device(s) 130. In some embodiments, the processor 210 may include one or more hardware processors, such as a microcontroller, a microprocessor, a reduced instruction set computer (RISC), an application-specific integrated circuits (ASICs), an application-specific instruction-set processor (ASIP), a central processing unit (CPU), a graphics processing unit (GPU), a physics processing unit (PPU), a microcontroller unit, a digital signal processor (DSP), a field-programmable gate array (FPGA), an advanced RISC machine (ARM), a programmable logic device (PLD), any circuit or processor capable of executing one or more functions, or the like, or any combinations thereof.

Merely for illustration, only one processor is described in the computing device 200. However, it should be noted that the computing device 200 in the present disclosure may also include multiple processors. Thus operations and/or method steps that are performed by one processor as described in the present disclosure may also be jointly or separately performed by the multiple processors. For example, if in the present disclosure the processor of the computing device 200 executes both operation A and operation B, it should be understood that operation A and operation B may also be performed by two or more different processors jointly or separately in the computing device 200 (e.g., a first processor executes operation A and a second processor executes operation B, or the first and second processors jointly execute operations A and B).

The storage device 220 may store data/information obtained from the imaging device 110, the terminal device(s) 130, the storage device 150, or any other component of the imaging system 100. In some embodiments, the storage device 220 may include a mass storage device, a removable storage device, a volatile read-and-write memory, a read-only memory (ROM), or the like, or any combination thereof. In some embodiments, the storage device 220 may store one or more programs and/or instructions to perform exemplary methods described in the present disclosure. For example, the storage device 220 may store a program for the processing device 140 for performing attenuation correction on a PET image.

The I/O 230 may input or output signals, data, and/or information. In some embodiments, the I/O 230 may enable user interaction with the processing device 140. In some embodiments, the I/O 230 may include an input device and an output device. Exemplary input devices may include a keyboard, a mouse, a touch screen, a microphone, or the like, or a combination thereof. Exemplary output devices may include a display device, a loudspeaker, a printer, a projector, or the like, or a combination thereof. Exemplary display devices may include a liquid crystal display (LCD), a light-emitting diode (LED)-based display, a flat panel display, a curved screen, a television device, a cathode ray tube (CRT), or the like, or a combination thereof.

The communication port 240 may be connected with a network (e.g., the network 120) to facilitate data communications. The communication port 240 may establish connections between the processing device 140 and the imaging device 110, the terminal device(s) 130, or the storage device 150. The connection may be a wired connection, a wireless connection, or a combination of both that enables data transmission and reception. The wired connection may include an electrical cable, an optical cable, a telephone wire, or the like, or any combination thereof. The wireless connection may include a Bluetooth network, a Wi-Fi network, a WiMax network, a WLAN, a ZigBee network, a mobile network (e.g., 3G, 4G, 5G, etc.), or the like, or any combination thereof. In some embodiments, the communication port 240 may be a standardized communication port, such as RS232, RS485, etc. In some embodiments, the communication port 240 may be a specially designed communication port. For example, the communication port 240 may be designed in accordance with the digital imaging and communications in medicine (DICOM) protocol.

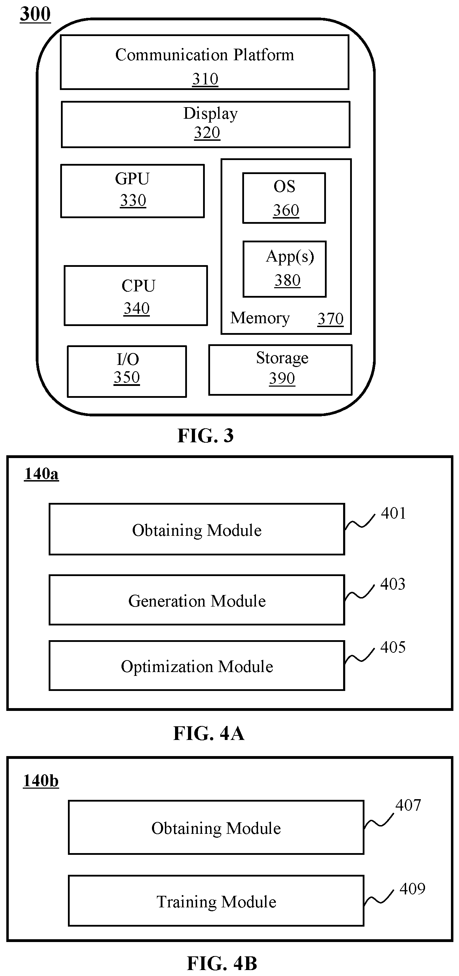

FIG. 3 is a schematic diagram illustrating hardware and/or software components of an exemplary mobile device 300 according to some embodiments of the present disclosure. In some embodiments, one or more components (e.g., a terminal device 130 and/or the processing device 140) of the imaging system 100 may be implemented on the mobile device 300.

As illustrated in FIG. 3, the mobile device 300 may include a communication platform 310, a display 320, a graphics processing unit (GPU) 330, a central processing unit (CPU) 340, an I/O 350, a memory 360, and a storage 390. In some embodiments, any other suitable component, including but not limited to a system bus or a controller (not shown), may also be included in the mobile device 300. In some embodiments, a mobile operating system 370 (e.g., iOS, Android, Windows Phone, etc.) and one or more applications 380 may be loaded into the memory 360 from the storage 390 in order to be executed by the CPU 340. The applications 380 may include a browser or any other suitable mobile apps for receiving and rendering information relating to image processing or other information from the processing device 140. User interactions with the information stream may be achieved via the I/O 350 and provided to the processing device 140 and/or other components of the imaging system 100 via the network 120.

To implement various modules, units, and their functionalities described in the present disclosure, computer hardware platforms may be used as the hardware platform(s) for one or more of the elements described herein. The hardware elements, operating systems and programming languages of such computers are conventional in nature, and it is presumed that those skilled in the art are adequately familiar therewith to adapt those technologies to generate an image as described herein. A computer with user interface elements may be used to implement a personal computer (PC) or another type of work station or terminal device, although a computer may also act as a server if appropriately programmed. It is believed that those skilled in the art are familiar with the structure, programming and general operation of such computer equipment and as a result, the drawings should be self-explanatory.

FIG. 4A and FIG. 4B are block diagrams illustrating exemplary processing devices 140a and 140b according to some embodiments of the present disclosure. In some embodiments, the processing devices 140a and 140b may be embodiments of the processing device 140 as described in connection with FIG. 1. In some embodiments, the processing devices 140a and 140b may be respectively implemented on a processing unit (e.g., the processor 210 illustrated in FIG. 2 or the CPU 340 as illustrated in FIG. 3). Merely by way of example, the processing devices 140a may be implemented on a CPU 340 of a terminal device, and the processing device 140b may be implemented on a computing device 200. Alternatively, the processing devices 140a and 140b may be implemented on a same computing device 200 or a same CPU 340. For example, the processing devices 140a and 140b may be implemented on a same computing device 200.

As illustrated in FIG. 4A, the processing device 140a may include an obtaining module 401, a generation module 403, and an optimization module 405.

The obtaining module 401 may be configured to obtain data/information relating to image optimization from one or more components of the imaging system 100. For example, the obtaining module 401 may obtain an initial image of a target object to be optimized, a reference image associated with the target object, and/or an optimized image from a storage device (e.g., the storage device 150, the storage device 220, etc.). As another example, the obtaining module 401 may obtain image/scan data of the target object from the storage device. The obtaining module 401 may reconstruct the initial image of the target object based on the scan data according to an image reconstruction algorithm. More descriptions regarding the obtaining operation may be found elsewhere in the present disclosure (e.g., operations 501 and 503 and relevant descriptions thereof).

The generation module 403 may be configured to generate a correlation reference image based on the reference image. For example, the generation module 403 may generate the correlation reference image by performing one or more processing operations on the reference image, more descriptions of which may be found elsewhere in the present disclosure (e.g., operation 505 and relevant description thereof).

The determination module 405 may be configured to determine an optimized image of the initial image by inputting the initial image, the reference image, and the correlation reference image into the optimization model. The optimization model may refer to a machine learning model such as a deep learning network, or a model that is configured for high-resolved and noise-reduced reconstruction. More descriptions regarding the determination of the optimized image may be found elsewhere in the present disclosure (e.g., operation 507 and relevant description thereof).

As illustrated in FIG. 4B, the processing device 140b may include an obtaining module 407 and a training module 409.

The obtaining module 407 may be configured to obtain data/information for model training. For example, the obtaining module 407 may obtain a plurality of training sampling each of which includes a sample image of a sample object, a sample reference image associated with the sample object, and a sample gold standard image corresponding to the sample image. As another example, the obtaining module 407 may obtain an initial machine learning model for training an optimized model. More descriptions regarding the obtaining of the plurality of training samples and/or the initial machine learning model may be found elsewhere in the present disclosure (e.g., operations 701 and 703 and relevant descriptions thereof).

The training module 409 may be configured to generate the optimized image by training, using the plurality of training samples, the initial machine learning model according to a training process. The training process may include for each of the plurality of training samples, determining a sample correlation reference image based on the sample reference image, and generating the optimization model using each of the plurality of training samples and a corresponding sample correlation reference image. More descriptions regarding the training of the optimized model may be found elsewhere in the present disclosure (e.g., operations 705 and 801-805 and relevant descriptions thereof).

It should be noted that the above description is merely provided for the purposes of illustration, and not intended to limit the scope of the present disclosure. Apparently, for persons having ordinary skills in the art, multiple variations and modifications may be conducted under the teachings of the present disclosure. However, those variations and modifications do not depart from the scope of the present disclosure. Each of the modules described above may be a hardware circuit that is designed to perform certain actions, e.g., according to a set of instructions stored in one or more storage media, and/or any combination of the hardware circuit and the one or more storage media. In some embodiments, the processing device 140a and/or the processing device 140b may share two or more of the modules, and any one of the modules may be divided into two or more units. For instance, the processing devices 140a and 140b may share a same acquisition module, that is, the obtaining module 401 and the obtaining module 407 are a same module. In some embodiments, the processing device 140a and/or the processing device 140b may include one or more additional modules, such as a storage module (not shown) for storing data. In some embodiments, the processing device 140a and the processing device 140b may be integrated into a same processing device.

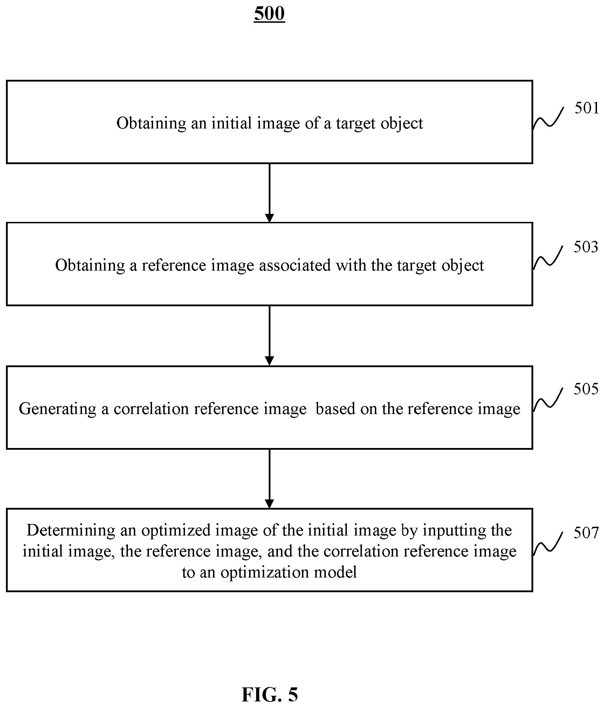

FIG. 5 is a flowchart illustrating an exemplary process for image optimization according to some embodiments of the present disclosure. In some embodiments, process 500 may be implemented as a set of instructions (e.g., an application) stored in a storage device (e.g., the storage device 150, the storage device 220, and/or the storage 390). The processing device 140a (e.g., the processor 210, the CPU 340, and/or one or more modules illustrated in FIG. 4A) may execute the set of instructions, and when executing the instructions, the processing device 140a may be configured to perform the process 500. The operations of the illustrated process presented below are intended to be illustrative. In some embodiments, the process 500 may be accomplished with one or more additional operations not described and/or without one or more of the operations discussed. Additionally, the order of the operations of process 500 illustrated in FIG. 5 and described below is not intended to be limiting.

In 501, the processing device 140a (e.g., the obtaining module 401) may obtain an initial image of a target object. The target object may be biological or non-biological. For example, the target object may include a patient, a man-made object, etc., as described elsewhere in the present disclosure.

In some embodiments, the initial image of the target object may be a medical image (e.g., an MRI image, a CT image, etc.) that does not satisfy a clinical need and needs to be optimized. For example, the initial image of the target object may be with a low image resolution and/or a low SNR. In some embodiments, the initial image of the target object may have a first image quality. For example, the first image quality may be less than or equal to a clinically desired image quality. For example, the initial image of the target object may be generated based on MR signals received by a VTC of an imaging device (e.g., the imaging device 110 such as an MRI device). As another example, the initial image of the target object may be acquired by an imaging device that is with a low spatial resolution.

In some embodiments, the initial image of the target object may be previously generated and stored in a storage device (e.g., the storage device 150, the storage device 220, etc.), and the processing device 140a may retrieve the initial image of the target object from the storage device. Alternatively, the initial image of the target object may be generated by the processing device 140a. For example, an imaging device (e.g., the imaging device 110) may be directed to perform a scan on the target object to acquire scan data of the target object. The processing device 140a may reconstruct the initial image of the target object based on the scan data according to an image reconstruction algorithm. Exemplary image reconstruction algorithms may include a 2-dimensional Fourier transform technique, a back projection technique (e.g., a convolution back projection technique, a filtered back projection technique), an iterative reconstruction technique, or the like, or any combination thereof.

In 503, the processing device 140a (e.g., the obtaining module 401) may obtain a reference image associated with the target object.

In some embodiments, the reference image associated with the target object may be a medical image with high image quality. For example, the reference image may have a second image quality higher than the first image quality. In some embodiments, the reference image associated with the target object may have clearer and/or finer structural information than the initial image. In some embodiments, the reference image associated with the target object may include a previous image of the target object or of another object (i.e., an object other than the target object). The object other than the target object may be similar to the target object. That is, the object may have similar profile information (e.g., the gender, the age, the weight, the height, etc.) and/or medical condition as the target object. For instance, the object may have the same gender, the same age (or substantially the same age), the same weight (or a substantially same weight), and/or the same height (or a substantially same height) as the target object. As used herein, substantially, when used to qualify a feature (e.g., equivalent to), indicates that the deviation from the feature is below a threshold, e.g., 30%, 25%, 20%, 15%, 10%, 5%, etc. For instance, a substantially same age/weight/height refers to an age/weight/height that is plus or minus (±) a corresponding threshold. As another example, the object other than the target object may have a same medical condition to be treated (e.g., breast cancer, lung cancer, etc.) as the target object.

In some embodiments, the reference image may be acquired by an imaging device (e.g., the imaging device 110) that is the same as or different from the imaging device that acquires the initial image. For example, the reference image may be acquired by the same imaging device as the initial image. The initial image may be generated using the VTC of the imaging device, while the reference image may be generated by MR signals acquired using surface coil(s) of the imaging device. As another example, the reference image and the initial image may be acquired by different imaging devices. The initial image may be acquired by an imaging device with a low spatial resolution, while the reference image may be acquired by an imaging device with a high spatial resolution. In some embodiments, the reference image may be acquired using the same imaging sequence as the initial image. For example, the initial image may be acquired using a T1 sequence, and the reference image may be acquired using the T1 sequence. As another example, the initial image may be acquired using a T2 sequence, and the reference image may be acquired using the T2 sequence. In some embodiments, the reference image may correspond to the same part/region as the initial image. For example, when the initial image is an MR image of the chest of a target patient, the reference image may be a previous MR image of the chest of the target patient or an MR image of the chest of a patient that is similar to the target patient. As another example, when the initial image is an MR image of the head of a target patient, the reference image may be a previous MR image of the head of the target patient or an MR image of the head of a patient that is similar to the target patient.

In some embodiments, the reference image of the target object may be previously stored in a storage device (e.g., the storage device 150, the storage device 220, etc.), and the processing device 140a may retrieve the reference image from the storage device. For example, the storage device may store a reference image pool including a plurality of reference images. The processing device 140a may determine whether there is a previous image of the target object with high image quality in the reference image pool. In response to determining that there is a previous image of the target object with high image quality in the reference image pool, the processing device 140a may obtain the image of the target object with high image quality as the reference image associated with the target object. In response to determining that there is no previous image of the target object with high image quality in the reference image pool, the processing device 140a may obtain an image of an object that is similar to the target object from the reference image pool and determine the image of the object that is similar to the target object as the reference image associated with the target object.

In 505, the processing device 140a (e.g., the generation module 403) may generate a correlation reference image based on the reference image.

In some embodiments, the correlation reference image may have a third image quality lower than the second image quality. For example, the third image quality may be consistent with the first image quality (e.g., a difference between the third image quality and the first image quality being below than a threshold). As another example, the correlation reference image may be with lower image resolution and/or higher noise than the reference image. In some embodiments, the correlation reference image may have same fine features (e.g., deep features, shallow features such as texture features, etc.) in the high-dimensional space as the reference image.

In some embodiments, the processing device 140a may generate the correlation reference image by performing one or more processing operations on the reference image for ensuring the third image quality of the correlation reference image may be consistent with the first image quality of the initial image, such that the initial image can be further processed with the correlation reference image. The one or more operations may not destroy the fine features of the reference image in the high-dimensional space. The one or more processing operations may include at least one processing operation of a downsampling operation, an upsampling operation, a noise-adding operation, or a filtering operation. Merely by way of example, the processing device 140a may generate a downsampled image by performing a downsampling operation on the reference image. The downsampled image may be with an image resolution that is lower than the image resolution of the reference image. The processing device 140a may generate an upsampled image by performing an upsampling operation on the downsampled image. The upsampled image may be with an image resolution the same as the reference image. The processing device 140a may generate the correlation reference image by performing a noise-adding operation on the upsampled image. For instance, the processing device 140a may add one or more noises to the upsampled image. The one or more noises may include white noise, a Gaussian noise, a Rician noise, a noise that is noncentral chi-square distribution, or the like, or any combination thereof. In some embodiments, parameters such as the degree of the downsampling and/or upsampling and the type and/or amount of the one or more noises may be a default setting or adjustable according to different situations (e.g., according to user experiences or automatically). For example, the processing device 140a may determine the degree of the downsampling and the degree of the upsampling according to user experience. As another example, the processing device 140a may determine the the degree of the downsampling and the degree of the upsampling by analyzing the initial image. As still another example, the processing device 140a may determine the type of the one or more noises according to the type of the optimized model. Different types of the optimization model may correspond to different types of noises. As a further example, the processing device 140a may determine the type of the one or more noises by analyzing noise(s) in the initial image. As further another example, the processing device 140a may determine the type of the one or more noises based on the type of the initial image. For instance, if the initial image is an MR image, the processing device 140a may determine the type of the one or more noises based on classic noises of an MR image. Alternatively, parameters such as the degree of the downsampling and/or upsampling and the type and/or amount of the one or more noises may be determined during the training of an optimization model to be used in operation 507. In some embodiments, the processing device 140a may generate, by performing the one or more processing operations on the reference image, the correlation reference image without impairing minutia features (e.g., deep features) of the reference image in the high-dimensional space. That is, the correlation reference image may retain the minutia features of the reference image.

In 507, the processing device 140a (e.g., the obtaining module 401, the optimization module 405, etc.) may determine an optimized image of the initial image by inputting the initial image, the reference image, and the correlation reference image to an optimization model

In some embodiments, the optimized image of the initial image refers to an image that is generated by performing, based on the reference image, an optimization operation on the initial image using the optimization model. The optimization image of the initial image may have a fourth image quality higher than the first image quality. For example, the optimized image may be with higher image resolution and/or lower noises than the initial image.

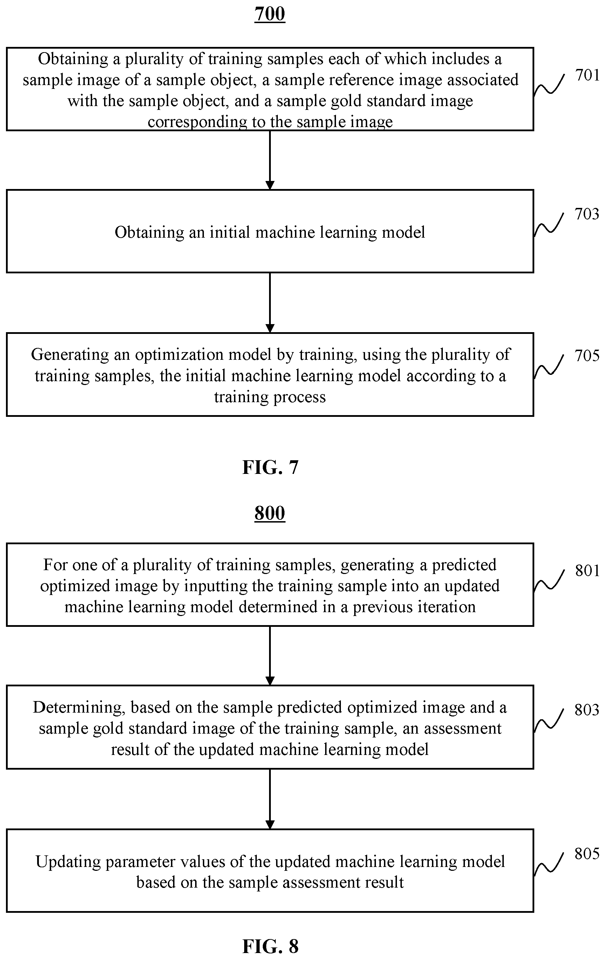

As used herein, the optimization model refers to a machine learning model such as a deep learning network that is configured for high-resolved and noise-reduced reconstruction using priori information existing in the refence image. In some embodiments, the optimization model may be of any type of deep learning network or model. For example, the optimization model may include a trained neural network model such as a trained convolutional neural network (CNN) model, a trained generative adversarial network (GAN) model, or any other suitable type of model. In some embodiments, the processing device 140a (e.g., the obtaining module 401) may obtain the optimization model from one or more components of the imaging system 100 (e.g., the storage device 150, the terminals(s) 130) or an external source via a network (e.g., the network 120). For example, the optimization model may be previously trained by a computing device (e.g., the processing device 140b), and stored in a storage device (e.g., the storage device 150, the storage device 220, and/or the storage 390) of the imaging system 100. The processing device 140a may access the storage device and retrieve the optimization model. In some embodiments, the optimization model may be generated according to a machine learning algorithm. The machine learning algorithm may include but not be limited to an artificial neural network algorithm, a deep learning algorithm, a decision tree algorithm, an association rule algorithm, an inductive logic programming algorithm, a support vector machine algorithm, a clustering algorithm, a Bayesian network algorithm, a reinforcement learning algorithm, a representation learning algorithm, a similarity and metric learning algorithm, a sparse dictionary learning algorithm, a genetic algorithm, a rule-based machine learning algorithm, or the like, or any combination thereof. The machine learning algorithm used to generate the optimization model may be a supervised learning algorithm, a semi-supervised learning algorithm, an unsupervised learning algorithm, or the like. In some embodiments, the optimization model may be generated by a computing device (e.g., the processing device 140b) by performing a process (e.g., process 700) for generating an optimization model disclosed herein. More descriptions regarding the generation of the optimization model may be found elsewhere in the present disclosure. See, e.g., FIGs. 7-9 and relevant descriptions thereof.

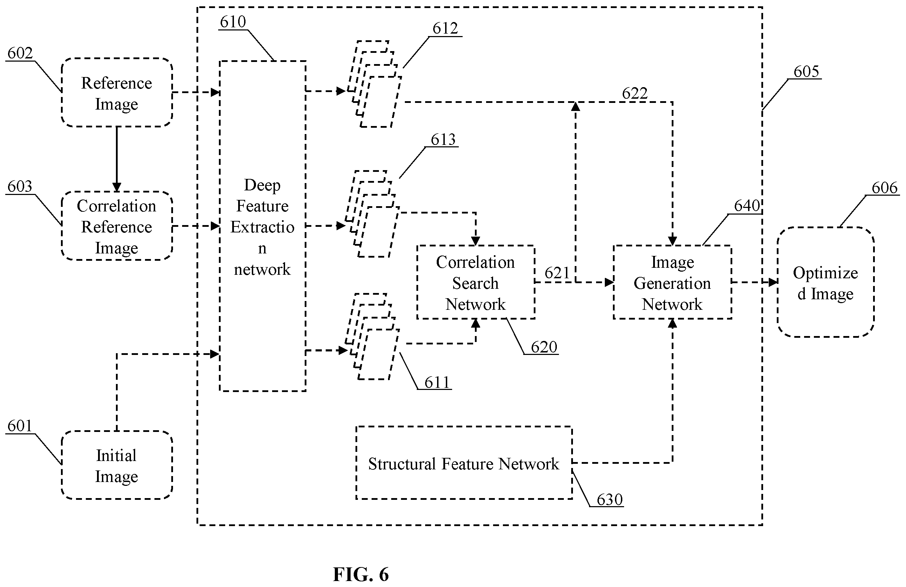

In some embodiments, the optimization model may be a trained deep learning model including a plurality of networks (also referred to as components) that are operably connected. For example, the plurality of components may be connected by multiple operations. The plurality of components may include a deep feature extraction network (also referred to as a deep feature extraction component), a structural feature extraction network (also referred to as a structural feature extraction component), a correlation search network (also referred to as a correlation search component), an image generation network (also referred to as an image generation component), or the like, or any combination thereof. The deep feature extraction component may be configured to extract multi-layer features from an image (e.g., the initial image, the reference image, and/or the correlation reference image). The structural feature extraction component may be configured to extract structural features from an image (e.g., the initial image). The correlation search component may be configured to construct a correspondence relationship between features (e.g., the multi-layer features and/or the structural features) of the initial image and features (e.g., the multi-layer features and/or the structural features) of the reference image. More descriptions regarding the connections and/or functions of the plurality of components of the optimization model may be found elsewhere in the present disclosure (e.g., FIG. 6 and the relevant description thereof).

In some embodiments, during the application of the optimization model, an input of the optimization model may include the initial image, the reference image, and the correlation reference image, and an output of the optimization model may include the optimized image. The plurality of components of the optimization model may perform, based on the reference image and the correlation reference image, the optimization operation on the initial image together. For example, the processing device 140a may directly input the initial image, the reference image, and the correlation reference image into the optimization model, and the optimization model may output the optimized image of the initial image. Alternatively, the processing device 140a may preprocess (e.g., perform a denoising operation, a normalization operation, etc., on) the initial image, the reference image, and/or the correlation reference image, and/or post-process (e.g., perform a denormalization operation on) an output of the optimization model to generate the optimized image of the initial image. More descriptions regarding the generation of the optimized image of a target object by application of the optimization model may be found elsewhere in the present disclosure (e.g., FIG. 6 and the description thereof).

In some embodiments, the processing device 140a may transmit the optimized image of the initial image to a terminal (e.g., a terminal device 130) for display. Optionally, a user (e.g., a doctor or an operator) of the terminal may input a response regarding the optimized image of the initial image via, for example, an interface of the terminal. For example, the user may evaluate whether the optimized image of the initial image satisfies a preset condition (e.g., a clinical need such as a clinically desired image quality). According to the evaluation result, the user may send a request to, for example, repeat or redo the optimization, to the processing device 140a.

It should be noted that the above description regarding the process 500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 500 may be omitted, and/or one or more additional operations may be added in the process 500. For example, a storing operation may be added elsewhere in the process 500. In the storing operation, the processing device 140a may store information and/or data associated with the image optimization process in a storage device (e.g., the storage device 150) disclosed elsewhere in the present disclosure. As another example, operation 505 may be omitted, and the optimized image of the initial image may be generated based on the reference image without the correlation reference image by application of the optimization model. In some embodiments, during the application of the optimization model, the input of the optimization model may include the initial image and the correlation reference image without the reference image, and the output of the optimization model may include the optimized image of the initial image. For example, the obtaining module 401 may obtain the initial image and the correlation reference image, and input them to the optimization model for outputting the optimized image. Alternatively, the input of the optimization model may include only the initial image, and the output of the optimization model may include the optimized image of the initial image.

FIG. 6 is a schematic diagram illustrating an application of an exemplary optimization model according to some embodiments of the present disclosure. The optimization model 605 provides an example of the optimization model as described in operation 507, which is not intended to limit the scope of the present disclosure. The description of the application of the optimization model 605 may be found elsewhere in the present disclosure. See, e.g., relevant discription in connection with the process 500 illustrated in FIG. 5. In some embodiments, the application of the optimization model 605 may be implemented by the processing device 140a as described in FIG. 5.

As shown in FIG. 6, the processing device 140a may obtain an initial image 601 of a target object. The initial image 601 may be similar to the initial image as described in operation 501. For example, the initial image 601 may be a medical image (e.g., an MR image) with a first image quality that does not satisfy the clinical need and needs to be optimized. The processing device 140a may obtain a reference image 602 associated with the target object. The reference image 602 may be similar to the reference image as described in operation 503. For example, the reference image 602 may be a previous image of the target object that is with a second image quality higher than the first image quality. The processing device 140a may generate a correlation reference image 603 based on the reference image 602. The correlation reference image 603 may be similar to the correlation reference image described in operation 505. For example, the processing device 140a may generate the correlation reference image 603 according to operation 505. The processing device 140a may generate an optimized image 606 of the initial image 601 by inputting the initial image 601, the reference image 602, and the correlation reference image 603 to the optimization model 605. The optimization model 605 may be similar to the optimization model that includes a plurality of components (i.e., networks) as described in operation 507. For illustration purposes in FIG. 6, the optimization model 605 may include a deep feature extraction network 610, a correlation search network 620, a structural feature extraction network 630, an image generation network 640, or the like, or any combination thereof.

The deep feature extraction network 610 may be configured to extract multi-layer features from an image (e.g., the initial image 601, the reference image 602, or the correlation reference image 603). The multi-layer features may include deep features and/or shallow features. As used herein, a deep feature of an image may include abstract information (e.g., semantic information) of the image. As used herein, a shallow feature of an image may include basic information (e.g., texture information, profile information such as color, shape, counter, etc.) of the image. In some embodiments, the deep feature extraction network 610 may be a multi-layer residual connection network that can extract the deep features and the shallow features, respectively, from an image to generate a feature map corresponding to the image. The feature map corresponding to the image may indicate the extracted deep features and the extracted shallow features of the image. That is, an input of the deep feature extraction network 610 may include one or more images, and an output of the deep feature extraction network 610 may include one or more feature maps corresponding to the one or more images respectively. For example, the processing device 140a may extract first multi-layer features from the initial image 601 to generate a first feature map 611. The processing device 140a may extract second multi-layer features from the reference image 602 to generate a second feature map 612. The processing device 140a may extract third multi-layer features from the correlation reference image 603 to generate a third feature map 613. The first multi-layer features, the second multi-layer features, and/or the third multi-layer features may include deep features and/or shallow features of its corresponding image.

The correlation search network 620 may be configured to construct a correspondance relationship between the features (the multi-layer features and/or the structural features) of the initial image and the features of the reference image. For example, as the correlation reference image 603 has an image quality consistent with the initial image 601, the correlation search network 620 may determine a correlation map 621 (not shown in FIG. 6) between the initial image 601 and the correlation reference image 603 based on the third feature map 613 corresponding to the correlation reference image 603 and the first feature map 611 corresponding to the initial image 601. That is, an input of the correlation search network 620 may include the first feature map 611 and the third feature map 613, and an output of the correlation search network 620 may include the correlation map 621. The correlation map 621 may indicate a correspondence relationship between the initial image 601 and the correlation reference image 603, which in turn may be used to indicate a correspondence relationship between the initial image 601 and the reference image 602.

The structural feature extraction network 630 may be configured to extract structural features from an image (e.g., the initial image). In some embodiments, the structural feature extraction network 630 may extract structural features from the initial image 601 to generate a fourth feature map (not shown in FIG. 6), e.g., by using multiple operation units (e.g., a residual connection unit, a dense connection unit, etc.) for removing noises which may obscure the structural features. That is, an input of the structural feature extraction network 630 may include the initial image 601, and an output of the structural feature extraction network 630 may include the fourth feature map that can indicate the structural features extracted from the initial image 601.

The image generation network 640 may be configured to generate an optimized image 606 of the initial image 601 by performing image reconstruction. In some embodiments, the image generation network 640 may be a deep learning network that can generate the optimized image 606 by performing image reconstruction based on the second feature map 612, the correlation map 621, and the fourth feature map. For example, an input of the image generation network 640 may include the second feature map 612, the correlation map 621, and the fourth feature map, and an output of the image generation network 640 may include the optimized image 606. In some embodiments, the second feature map may be processed before input to the image generation network 640 for improving the accuracy and/or the image quality of the optimized image 606. For example, the deep feature extraction network 610 may be operably connected with the image generation network 640 via one or more operations (e.g., an attention algorithm). The attention algorithm may be configured to fuse features (e.g., deep features of the second feature map 612 and the correlation map in multiple scales) to generate a correlated feature map 622 (not shown in FIG. 6). As used herein, features in multiple scales refer to features whose dimensions are in different orders. For instance, features in multiple scales may include features whose dimensions are in the order of millimeters and features whose dimensions are in the order of micrometers. One or more attention weights may be generated for features of interest (i.e., deep features) by using the attenuation algorithm. The correlated feature map 622 may include the one or more attention weights. The greater an attention weight is, the more attention may be paid to a feature corresponding to the attention weight during image reconstruction. In other words, a feature corresponding to a high attention weight may be more focused on during image reconstruction than a feature corresponding to a low attention weight. For instance, the second feature map 612 and the correlation map 621 may be processed (e.g., compared or searched) using the attenuation algorithm in a first scale (e.g., with respect to features whose dimensions are in a first order), a second scale (e.g., with respect to features whose dimensions are in a second order), etc., to determine the correlated feature map 622. Dimensions in the first order may be larger than the dimensions in the second order. Further, the image generation network 640 may perform image reconstruction by fusing features of the correlated feature map 622, the correlation map 621, and the fourth feature map to generate the optimized image 606. In such cases, an input of the image generation network 640 may include the correlation map 621, the fourth feature map, and the correlated feature map 622, and an output of the image generation network 640 may include the optimized image 606. During the image reconstruction, the correlated feature map 622 may guide the image reconstruction to focus on the features in the high-dimensional space according to the corresponding one or more attention weights, and accordingly accurate local details and key structures can be transferred from the reference image 602 to the initial image 601 for generating the optimized image 606.

It should be noted that the above description regarding the optimization model 605 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more networks or components may be added to the optimization model 605 and/or one or more networks or components of the optimization model 605 may be omitted. In some embodiments, a network or component may be added for generating the correlation reference image 603 based on the reference image 602. In such cases, the input of the optimization model 605 may include only the initial image 601 and the reference image 602 without the correlation reference image 603.

FIG. 7 is a flowchart illustrating an exemplary process 700 for generating an optimization model according to some embodiments of the present disclosure. In some embodiments, process 700 may be implemented as a set of instructions (e.g., an application) stored in a storage device (e.g., the storage device 150, storage device 220, and/or storage 390). The processing device 140b (e.g., the processor 210, the CPU 340, and/or one or more modules illustrated in FIG. 4B) may execute the set of instructions, and when executing the instructions, the processing device 140b may be configured to perform the process 700. The operations of the illustrated process presented below are intended to be illustrative. In some embodiments, the process 700 may be accomplished with one or more additional operations not described and/or without one or more of the operations discussed. Additionally, the order of the operations of process 700 illustrated in FIG. 7 and described below is not intended to be limiting. In some embodiments, the optimization model described in operation 503 in FIG. 5 or the optimization model 605 described in FIG. 6 may be obtained according to the process 700. In some embodiments, the process 700 may be performed by another device or system other than the imaging system 100, e.g., a device or system of a vendor of a manufacturer. For illustration purposes, the implementation of the process 700 by the processing device 140b is described as an example.

In 701, the processing device 140b (e.g., the obtaining module 407) may obtain a plurality of training samples. A training sample may include a sample image of a sample object, a sample reference image associated with the sample object, and a sample gold standard image corresponding to the sample image.

As used herein, a sample object refers to an object whose images are used for training the optimization model. The sample object may possess one or more characteristics as the subject as described in connection with FIG. 5. Merely by way of example, if the optimization model is used to perform image optimization on an image of the head of a patient (or a portion thereof), the sample object may be the head of another patient (or a portion thereof). As used herein, a sample image of a sample object refers to a sample image that is used for training the optimization model, and a sample gold standard image corresponding to a sample image refers to a sample image that is used as ground truth for the sample image. In some embodiments, for a training sample of the plurality of training samples, the sample image may have a first image quality, the sample reference image may have a second image quality higher than the first image quality, and the sample gold standard image may have a third image quality higher than the first image quality. For instance, for a training sample, the sample image may be generated based on MR signals received by a VTC of an imaging device or acquired by an imaging device with a low spatial resolution; the sample reference image and/or the sample gold standard image of the training sample may be generated based on MR signals received by surface coil(s) of the imaging device or acquired by an imaging device with a high spatial resolution. In some embodiments, the second image quality may be consistent with the third image quality (e.g., a difference between the second image quality and the third image quality being below a threshold). In some embodiments, for a training sample, the sample image, the sample reference image, and the sample gold standard image of the training sample may be acquired using a same imaging sequence (e.g., T1 sequence, T2 sequence, etc.).

In some embodiments, for a training sample, a sample reference image associated with a sample object and/or the sample gold standard image corresponding to the sample image may include a previous image of the sample object or an image of another sample object that is other than the sample object, which is similar to the description of the reference image associated with the target object. The other sample object may be similar to the sample object, e.g., having similar profile information as the sample object. For example, each of the sample reference image and the sample gold standard image may include a previous image of the sample object. As another example, the sample reference image may include a sample image of another sample object that is other than the sample object, and the sample gold standard image may include a previous image of the sample object. In some embodiments, the sample reference image and the sample gold standard image of a same training sample may be acquired using the same imaging device under the same imaging condition. For example, for a training sample, the sample reference image and the sample gold standard image may be a same image. As another example, for a training sample, the sample reference image may be acquired by performing, using first scan parameters, a first scan on a sample object by an imaging device, and the sample gold standard image may be acquired, using the first scan parameters, a second scan on the same sample object by the same imaging device. By the acquisition of the sample reference image and the sample gold standard image of the same training sample according to different scans under the same imaging condition, the plurality of training samples may have randomness in terms of imaging quality, which may further improve the accuracy of the optimization model.

In some embodiments, a training sample may be previously generated and stored in a storage device (e.g., the storage device 150, the storage device 220, the storage 390, or an external database). The processing device 140b may retrieve the training sample directly from the storage device. In some embodiments, at least a portion of a training sample may be generated by the processing device 140b. Merely by way of example, for a training sample, the processing device 140b may obtain the sample image and the sample reference image from a storage device; the processing device 140b may determine the sample gold standard image based on the sample image using an image optimization technique other than the optimization model. As another example, for a training sample, the processing device 140b may obtain the sample reference image and the sample gold standard image from a storage device; the processing device 140b may determine one or more sample images based on the sample reference image or the sample gold standard image using an image processing technique including, e.g., downsampling, upsampling, noise-adding, filtering, etc.

In 703, the processing device 140b (e.g., the obtaining module 407) may obtain an initial machine learning model.

The initial machine learning model may be any type of machine learning model, which is similar to the optimized model as described in operation 507. For example, the initial machine learning model may include a plurality of components or networks such as a deep feature extraction component, a structural feature extraction component, a correlation search component, an image generation component, or the like, or any combination thereof, which is similar to the plurality of components of the optimization model 605 as described in FIG. 6. In some embodiments, the initial machine learning model may include one or more additional components, such as a skip-connection, a residual block, a dense block, or the like, or any combination thereof. Such additional components may be configured to combine different features extracted by different components or networks of the initial machine learning model, thereby accelerating convergence during model training and improving the accuracy of the resulting optimization model.

In some embodiments, the initial machine learning model may include one or more model parameters. The processing device 140b may initialize parameter value(s) of the model parameter(s) before training, and the value(s) of the model parameter(s) of the initial machine learning model may be updated during the training of the initial machine learning model. For illustration purposes, the initial machine learning model may be a deep learning model such as a neural network. Exemplary model parameters of the initial machine learning model may include a loss function, the number (or count) of convolutional layers, the number (or count) of kernels, a kernel size, a stride, a padding of each convolutional layer, or the like, or any combination thereof.

In 705, the processing device 140b (e.g., the training module 409) may generate the optimization model by training, using the plurality of training samples, the initial machine learning model according to a training process.

In some embodiments, the training process may include, for each of the plurality of training samples, determining a sample correlation reference image based on the sample reference image. The sample correction reference image may have a fourth image quality lower than the second image quality. For example, for a training sample, the processing device 140b may generate the sample correlation reference image by performing one or more operations on the sample reference image, which is similar to the generation of the correlation reference image based on the reference image as described in operation 505. The training process may also include generating the optimization model using each of the plurality of training samples and a corresponding sample correlation reference image.

In some embodiments, the components or networks (e.g., the deep feature extraction component, the structural feature extraction component, the correlation search component, the image generation component, etc.) of the initial machine learning model may be trained in parallel during the training process. That is, the plurality of components or networks of the initial machine learning model may be operably connected and updated as a whole. Merely by way of example, the processing device 140b may train the initial machine learning model by iteratively updating the model parameters of the initial machine learning model based on the plurality of training samples and corresponding sample correlation reference images.

In some embodiments, the training process may include one or more iterations. For an iteration of the plurality of iterations, the processing device 140b may, for one of the plurality of training samples, generate a sample predicted optimized image of the sample image by inputting the training sample (e.g., the sample image and the sample reference image of the training sample) to an updated machine learning model determined in a previous iteration. For example, the processing device 140b may generate the sample predicted optimized image by inputting a sample image, a sample reference image of the training sample, and a sample correlation reference image into the initial machine learning model (e.g., in a first iteration) or the updated machine learning model, in which sample correlation reference image may be determined based on the sample reference image. The processing device 140b may determine, based on the sample predicted optimized image and a sample gold standard image of the training sample, a sample assessment result of the updated machine learning model. The processing device 140b may update parameter values of the updated machine learning model based on the sample assessment result. More descriptions regarding the one or more iterations of the training process may be found elsewhere in the present disclosure (e.g., FIG. 8 and relevant descriptions thereof).

It should be noted that the above description regarding process 700 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations may be added to or omitted from the process 700. For example, a storage operation for storing the optimized model may be added after operation 705 for further use (e.g., in image optimization as described in connection with FIG. 5). As another example, after the optimization model is generated, the processing device 140b may further test the optimization model using a set of testing samples. Additionally or alternatively, the processing device 140b may update the optimization model periodically or irregularly based on one or more newly-generated training images (e.g., new samples generated in medical diagnosis).

FIG. 8 is a flowchart illustrating an exemplary training process according to some embodiments of the present disclosure. In some embodiments, process 800 may be implemented as a set of instructions (e.g., an application) stored in a storage device (e.g., the storage device 150, storage device 220, and/or storage 390). The processing device 140b (e.g., the processor 210, the CPU 340, and/or one or more modules illustrated in FIG. 4B) may execute the set of instructions, and when executing the instructions, the processing device 140b may be configured to perform process 800. In some embodiments, one or more operations of process 800 may be performed to achieve at least part of operation 705 as described in connection with FIG. 7. For example, the process 800 may be performed to achieve an iteration (e.g., a current iteration) in the training process as described in operation 705, during which the components of the initial machine learning model are trained in parallel. The current iteration may be performed based on at least some of the training samples. In some embodiments, a same set or different sets of training samples may be used in different iterations in the training process.

In 801, for one of a plurality of training samples, the processing device 140b (e.g., the training module 409) may generate a predicted optimized image by inputting the training sample (e.g., the sample image and the sample reference image of the training sample) to an updated machine learning model determined in a previous iteration.

During the application of the updated machine learning model on a training sample, the deep feature extraction component of the updated machine learning model may be configured to extract first sample multi-layer features from a sample image of the training sample to generate a first sample feature map; extract second sample multi-layer features from the sample reference image of the training sample to generate a second sample feature map; and extract third sample multi-layer features from a sample correlation reference image generated based on the sample reference image to generate a third sample feature map. At least one of the first sample multi-layer features, the second sample multi-layer features, or the third sample multi-layer features may include sample deep features and/or sample shallow features. The structural feature extraction component of the updated machine learning model may be configured to extract sample structural features from the sample image of the training sample to generate a fourth sample feature map. The correlation search component of the updated machine learning model may be configured to determine a sample correlation map between the sample image of the training sample and the corresponding sample correlation reference image based on the first sample feature map and the third sample feature map. The image generation component of the updated machine learning model may be configured to generate a sample predicted optimized image of the sample image of the training sample based on the second sample feature map, the sample correlation map, and the fourth sample feature map. In some embodiments, the deep feature extraction component may be operably connected with the image generation component via an attention algorithm. The attenuation algorithm may be configured to fuse features of the second sample feature map and the sample correlation map in multiple scales. For example, a sample correlated feature map may be generated, using the attenuation algorithm, based on the second sample feature map and the sample correlation map. In such cases, an input of the image generation component may include the fourth sample feature map, the sample correlation map, and the sample correlated feature map, and an output of the image generation component may include the sample predicted optimized image of the sample image of the training sample.

In 803, the processing device 140b (e.g., the training module 409) may determine, based on the sample predicted optimized image and a sample gold standard image of the training sample, an assessment result of the updated machine learning model.

The sample assessment result may indicate the accuracy and/or efficiency of the updated machine learning model. In some embodiments, the sample assessment result may be associated with a difference between the sample predicted optimized image and the sample gold standard image of the training sample. For example, a value of a loss function may be determined to measure a difference between the sample predicted optimized image and the sample gold standard image of the training sample. The processing device 140b may determine the sample assessment result based on the value of the loss function. As another example, a value of an overall loss function may be determined to measure an overall difference between the sample predicted optimized image and the sample gold standard image of each of the plurality of training samples (i.e., the training sample in operation 801 and other training samples of the plurality of training samples). The processing device 140b may determine the sample assessment result based on the value of the overall loss function. In some embodiments, the sample assessment result may be associated with the time needed for the updated machine learning model to generate the sample predicted optimized image of the training sample. For example, the short the needed time is, the high efficiency the updated machine learning model may have.

In some embodiments, the sample assessment result may include a determination as to whether a termination condition is satisfied in the current iteration. In some embodiments, the termination condition may relate to the value of the loss function and/or the overall loss function in the updated machine learning model. For example, the termination condition may be satisfied if the value of the loss function and/or the overall loss function is minimal or smaller than a threshold (e.g., a constant). As another example, the termination condition may be satisfied if the value of loss function and/or the overall loss function converges. In some embodiments, convergence may be deemed to have occurred if, for example, the variation of the values of the loss function and/or the overall loss function in two or more consecutive iterations is equal to or smaller than a threshold (e.g., a constant), a certain count of iterations may be performed, or the like. In some embodiments, the termination condition may be satisfied if the time needed for the updated machine learning model to generate the sample predicted optimized image of the training sample is smaller than a threshold.

In 805, the processing device 140b (e.g., the training module 409) may update parameter values of the updated machine learning model based on the sample assessment result.

In some embodiments, the processing device 140b may determine whether the termination condition is satisfied based on the sample assessment result. In response to a determination that the termination condition is satisfied, the processing device 140b may determine the updated machine learning model as the optimization model. In response to a determination that the termination condition is not satisfied, the processing device 140b may update the parameter values of the updated machine learning model to be used in a next iteration based on the sample assessment result.

It should be noted that the above descriptions regarding the process 800 are merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. The operations of the illustrated process presented above are intended to be illustrative. In some embodiments, the process 800 may be accomplished with one or more additional operations not described and/or without one or more of the operations discussed.

## Claims

1. A method for image optimization, the method being implemented by a computing device (200), the method comprising:
obtaining an initial image of a target object, the initial image having a first image quality;
obtaining a correlation reference image that is generated based on a reference image associated with the target object, the reference image having a second image quality higher than the first image quality, and the correlation reference image having a third image quality lower than the second image quality;
determining an optimized image of the initial image by inputting the initial image and the correlation reference image to an optimization model, wherein the optimization model refers to a machine learning model that is configured for high-resolved and noise-reduced reconstruction using priori information existing in the reference image, and the optimized image has a fourth image quality higher than the first image quality, wherein the optimization model includes a deep feature extraction component and a structural feature extraction component, the deep feature extraction component is configured to:
extract first multi-layer features from the initial image to generate a first feature map;
extract second multi-layer features from the reference image to generate a second feature map;
extract third multi-layer features from the correlation reference image to generate a third feature map; and
the structural feature extraction component is configured to extract structural features from the initial image to generate a fourth feature map.

2. The method of claim 1, wherein at least one of the first multi-layer features, the second multi-layer features, or the third multi-layer features include deep features and/or shallow features.

3. The method of claim 1 or 2, wherein the optimization model includes a correlation search component configured to determine a correlation map between the initial image and the correlation reference image based on the first feature map and the third feature map.

4. The method of claim 3, wherein the optimization model further includes an image generation component configured to generate the optimized image based on the second feature map, the correlation map, and the fourth feature map.

5. The method of claim 4, wherein the deep feature extraction component is operably connected with the image generation component via an attention algorithm; and
the method further comprises:
generating a correlated feature map, using the attention algorithm, based on the second feature map and the correlation map, wherein
an input of the image generation component includes the fourth feature map, the correlation map, and the correlated feature map, and
an output of the image generation component includes the optimized image.

6. The method of claim 5, wherein the attention algorithm is configured to fuse features of the second feature map and the correlation map in multiple scales.

7. The method of any one of claims 1 to 6, wherein the optimization model is trained using a plurality of training samples, and
each of at least one of the plurality of training samples includes a sample image of a sample object and a sample reference image of the sample object; preferably
the each of at least one of the plurality of training samples includes a sample image of a first sample object and a sample reference image of a second sample object that is other than the first sample object.

8. The method of claim 1, wherein the optimization model is generated by:
obtaining a plurality of training samples each of which includes a sample image of a sample object, a sample reference image associated with the sample object, and a sample gold standard image corresponding to the sample image, the sample image having a first image quality, the sample reference image having a second image quality higher than the first image quality, and the sample gold standard image having a third image quality higher than the first image quality;
obtaining an initial machine learning model; and
generating the optimization model by training, using the plurality of training samples, the initial machine learning model according to a training process including:
for each of the plurality of training samples, determining a sample correlation reference image based on the sample reference image, the sample correlation reference image having a fourth image quality lower than the second image quality; and
generating the optimization model using each of the plurality of training samples and a corresponding sample correlation reference image.

9. The method of claim 8, wherein a deep feature extraction component, a structural feature extraction component, a correlation search component, and an image generation component of the initial machine learning model are trained in parallel.

10. The method of claim 9, wherein for each of the plurality of training samples and a corresponding sample correlation reference image,
the deep feature extraction component of the initial machine learning model is configured to
extract first sample multi-layer features from a sample image of the training sample to generate a first sample feature map;
extract second sample multi-layer features from the sample reference image of the training sample to generate a second sample feature map; and
extract third sample multi-layer features from the corresponding sample correlation reference image to generate a third sample feature map; preferably
at least one of the first sample multi-layer features, the second sample multi-layer features, or the third sample multi-layer features includes sample deep features and/or sample shallow features;
the structural feature extraction component of the initial machine learning model is configured to extract sample structural features from the sample image of the training sample to generate a fourth sample feature map;
the correlation search component of the initial machine learning model is configured to determine a sample correlation map between the sample image of the training sample and the corresponding sample correlation reference image based on the first sample feature map and the third sample feature map; and
the image generation component of the initial machine learning model is configured to generate a sample predicted optimized image of the sample image of the training sample based on the second sample feature map, the sample correlation map, and the fourth sample feature map.

11. The method of any one of claims 8 to 10, wherein the training process includes a plurality of iterations, each of the plurality of iterations including:
for one of the plurality of training samples,
generating a sample predicted optimized image by inputting the training sample into an updated machine learning model determined in a previous iteration;
determining, based on the sample predicted optimized image and a sample gold standard image of the training sample, a sample assessment result of the updated machine learning model; and
updating parameter values of the updated machine learning model based on the sample assessment result.

12. The method of claim 11, wherein the sample assessment result is determined based on at least one of:
a difference between the sample predicted optimized image of the training sample and the sample gold standard image of the training sample, or
a time needed for the updated machine learning model to generate the sample predicted optimized image of the training sample.

13. A system (100) for image optimization, the system comprising:
a storage device (150) including a set of instructions;
at least one processor (210) in communication of the storage device (150), wherein when executing the set of instructions, the at least one processor (210) is configured to direct the system (100) to perform operations including:
obtaining an initial image of a target object, the initial image having a first image quality;
obtaining a correlation reference image that is generated based on a reference image associated with the target object, the reference image having a second image quality higher than the first image quality, and the correlation reference image having a third image quality lower than the second image quality;
determining an optimized image of the initial image by inputting the initial image and the correlation reference image to an optimization model, wherein the optimization model refers to a machine learning model that is configured for high-resolved and noise-reduced reconstruction using priori information existing in the reference image, and the optimized image has a fourth image quality higher than the first image quality, wherein the optimization model includes a deep feature extraction component and a structural feature extraction component, the deep feature extraction component is configured to:
extract first multi-layer features from the initial image to generate a first feature map;
extract second multi-layer features from the reference image to generate a second feature map;
extract third multi-layer features from the correlation reference image to generate a third feature map; and
the structural feature extraction component is configured to extract structural features from the initial image to generate a fourth feature map.

14. The system (100) of claim 13, wherein at least one of the first multi-layer features, the second multi-layer features, or the third multi-layer features include deep features and/or shallow features.

15. The system (100) of claim 13 or 14, wherein the optimization model includes a correlation search component configured to determine a correlation map between the initial image and the correlation reference image based on the first feature map and the third feature map.

## Patentansprüche

1. Verfahren zur Bildoptimierung, wobei das Verfahren durch eine Rechenvorrichtung (200) implementiert wird, wobei das Verfahren umfasst:
Erhalten eines Ausgangsbilds eines Zielobjekts, wobei das Ausgangsbild eine erste Bildqualität aufweist;
Erhalten eines Korrelationsreferenzbilds, das basierend auf einem Referenzbild erzeugt wird, das mit dem Zielobjekt verknüpft ist, wobei das Referenzbild eine zweite Bildqualität aufweist, die höher ist als die erste Bildqualität, und das Korrelationsreferenzbild eine dritte Bildqualität aufweist, die niedriger ist als die zweite Bildqualität;
Bestimmen eines optimierten Bilds des Ausgangsbilds durch Eingeben des Ausgangsbilds und des Korrelationsreferenzbilds in ein Optimierungsmodell, wobei sich das Optimierungsmodell auf ein maschinelles Lernmodell bezieht, das für hochauflösende und rauschreduzierte Rekonstruktion unter Verwendung von im Referenzbild vorhandenen Vorinformationen konfiguriert ist, und das optimierte Bild eine vierte Bildqualität aufweist, die höher ist als die erste Bildqualität, wobei das Optimierungsmodell eine Komponente zur Extraktion tiefer Merkmale und eine Komponente zur Extraktion struktureller Merkmale beinhaltet, wobei die Komponente zur Extraktion tiefer Merkmale konfiguriert ist für:
Extrahieren erster mehrschichtiger Merkmale aus dem Ausgangsbild, um eine erste Merkmalskarte zu erzeugen;
Extrahieren zweiter mehrschichtiger Merkmale aus dem Referenzbild, um eine zweite Merkmalskarte zu erzeugen;
Extrahieren dritter mehrschichtiger Merkmale aus dem Korrelationsreferenzbild, um eine dritte Merkmalskarte zu erzeugen; und
wobei die Komponente zur Extraktion struktureller Merkmale dazu konfiguriert ist, aus dem Ausgangsbild strukturelle Merkmale zu extrahieren, um eine vierte Merkmalskarte zu erzeugen.

2. Verfahren nach Anspruch 1, wobei mindestens eines der ersten mehrschichtigen Merkmale, der zweiten mehrschichtigen Merkmale oder dritten mehrschichtigen Merkmale tiefe Merkmale und/oder flache Merkmale beinhalten.

3. Verfahren nach Anspruch 1 oder 2, wobei das Optimierungsmodell eine Komponente zur Korrelationssuche beinhaltet, die dazu konfiguriert ist, eine Korrelationskarte zwischen dem Ausgangsbild und dem Korrelationsreferenzbild basierend auf der ersten Merkmalskarte und der dritten Merkmalskarte zu bestimmen.

4. Verfahren nach Anspruch 3, wobei das Optimierungsmodell ferner eine Bilderzeugungskomponente beinhaltet, die dazu konfiguriert ist, das optimierte Bild basierend auf der zweiten Merkmalskarte, der Korrelationskarte und der vierten Merkmalskarte zu erzeugen.

5. Verfahren nach Anspruch 4, wobei die Komponente zur Extraktion tiefer Merkmale über einen Aufmerksamkeitsalgorithmus mit der Bilderzeugungskomponente betriebsfähig verbunden ist; und
das Verfahren ferner umfasst:
Erzeugen einer korrelierten Merkmalskarte unter Verwendung des Aufmerksamkeitsalgorithmus basierend auf der zweiten Merkmalskarte und der Korrelationskarte, wobei
eine Eingabe der Bilderzeugungskomponente die vierte Merkmalskarte, die Korrelationskarte und die korrelierte Merkmalskarte beinhaltet, und
eine Ausgabe der Bilderzeugungskomponente das optimierte Bild beinhaltet.

6. Verfahren nach Anspruch 5, wobei der Aufmerksamkeitsalgorithmus dazu konfiguriert ist, Merkmale der zweiten Merkmalskarte und der Korrelationskarte in mehreren Skalen vereinigt.

7. Verfahren nach einem der Ansprüche 1 bis 6, wobei das Optimierungsmodell unter Verwendung einer Vielzahl von Trainingsproben trainiert wird, und
mindestens eine der Vielzahl von Trainingsproben jeweils ein Probenbild eines Probenobjekts und ein Probenreferenzbild des Probenobjekts beinhaltet; vorzugsweise
die jeweilige mindestens eine der Vielzahl von Trainingsproben ein Probenbild eines ersten Probenobjekts und ein Probenreferenzbild eines zweiten Probenobjekts beinhaltet, das ein anderes als das erste Probenobjekt ist.

8. Verfahren nach Anspruch 1, wobei das Optimierungsmodell erzeugt wird durch:
Erhalten einer Vielzahl von Trainingsproben, die jeweils ein Probenbild eines Probenobjekts, ein dem Probenobjekt zugeordnetes Probenreferenzbild und ein dem Probenbild entsprechendes Proben-Goldstandardbild beinhalten, wobei das Probenbild eine erste Bildqualität aufweist, das Probenreferenzbild eine zweite Bildqualität aufweist, die höher als die erste Bildqualität ist, und das Proben-Goldstandardbild eine dritte Bildqualität aufweist, die höher als die erste Bildqualität ist;
Erhalten eines maschinellen Ausgangslernmodells; und
Erzeugen des Optimierungsmodells durch Training, unter Verwendung der Vielzahl von Trainingsbeispielen, wobei das maschinelle Ausgangslernmodell gemäß einem Trainingsprozess beinhaltet:
für jede der Vielzahl von Trainingsproben, Bestimmen eines Probenkorrelationsreferenzbilds basierend auf dem Probenreferenzbild, wobei das Probenkorrelationsreferenzbild eine vierte Bildqualität aufweist, die niedriger ist als die zweite Bildqualität; und
Erzeugen des Optimierungsmodells unter Verwendung jeder der Trainingsproben und eines entsprechenden Probenkorrelationsreferenzbilds.

9. Verfahren nach Anspruch 8, wobei eine Komponente zur Extraktion tiefer Merkmale, eine Komponente zur Extraktion struktureller Merkmale, eine Komponente zur Korrelationssuche und eine Komponente zur Bilderzeugung des maschinellen Ausgangslernmodells parallel trainiert werden.

10. Verfahren nach Anspruch 9, wobei für jede der Trainingsproben und ein entsprechendes Probenkorrelationsreferenzbild
die Komponente zur Extraktion tiefer Merkmale des maschinellen Ausgangslernmodells konfiguriert ist zum
Extrahieren erster mehrschichtiger Probenmerkmale aus einem Probenbild der Trainingsprobe, um eine erste Probenmerkmalskarte zu erzeugen;
Extrahieren zweiter mehrschichtiger Probenmerkmale aus dem Probenreferenzbild der Trainingsprobe, um eine zweite Probenmerkmalskarte zu erzeugen; und
Extrahieren dritter mehrschichtiger Probenmerkmale aus dem entsprechenden Probenkorrelationsreferenzbild, um eine dritte Probenmerkmalskarte zu erzeugen; vorzugsweise
mindestens eines der ersten mehrschichtigen Probenmerkmale, der zweiten mehrschichtigen Probenmerkmale oder dritten mehrschichtigen Probenmerkmale tiefe Probenmerkmale und/oder flache Probenmerkmale beinhaltet;
die Komponente zur Extraktion struktureller Merkmale des maschinellen Ausgangslernmodells dazu konfiguriert ist, aus dem Probenbild der Trainingsprobe strukturelle Probenmerkmale zu extrahieren, um eine vierte Probenmerkmalskarte zu erzeugen;
die Komponente zur Korrelationssuche des maschinellen Ausgangslernmodells dazu konfiguriert ist, eine Probenkorrelationskarte zwischen dem Probenbild der Trainingsprobe und dem entsprechenden Probenkorrelationsreferenzbild basierend auf der ersten Probenmerkmalskarte und der dritten Probenmerkmalskarte zu bestimmen; und
die Komponente zur Bilderzeugung des maschinellen Ausgangslernmodells dazu konfiguriert ist, ein vorhergesagtes optimiertes Probenbild des Probenbilds der Trainingsprobe basierend auf der zweiten Probenmerkmalskarte, der Probenkorrelationskarte und der vierten Probenmerkmalskarte zu erzeugen.

11. Verfahren nach einem der Ansprüche 8 bis 10, wobei der Trainingsprozess eine Vielzahl von Iterationen beinhaltet, wobei jede der Vielzahl von Iterationen beinhaltet:
für eine der Vielzahl von Trainingsproben,
Erzeugen eines vorhergesagten optimierten Probenbilds durch Eingeben der Trainingsprobe in ein aktualisiertes maschinelles Lernmodell, das in einer vorherigen Iteration bestimmt wurde;
Bestimmen, basierend auf dem vorhergesagten optimierten Probenbild und einem Proben-Goldstandardbild der Trainingsprobe, eines Probenbewertungsergebnisses des aktualisierten maschinellen Lernmodells; und
Aktualisieren von Parameterwerten des aktualisierten maschinellen Lernmodells basierend auf dem Probenbewertungsergebnis.

12. Verfahren nach Anspruch 11, wobei das Probenbewertungsergebnis basierend auf mindestens einem bestimmt wird von:
einer Differenz zwischen dem vorhergesagten optimierten Probenbild der Trainingsprobe und dem Proben-Goldstandardbild der Trainingsprobe, oder
einer Zeit, die das aktualisierte maschinelle Lernmodell benötigt, um das vorhergesagte optimierte Probenbild der Trainingsprobe zu erzeugen.

13. System (100) zur Bildoptimierung, wobei das System umfasst:
eine Speichervorrichtung (150), die einen Satz von Anweisungen beinhaltet;
mindestens einen Prozessor (210) in Kommunikation mit der Speichervorrichtung (150), wobei, wenn der Satz von Anweisungen ausgeführt wird, der mindestens eine Prozessor (210) dazu konfiguriert ist, das System (100) anzuleiten, Operationen durchzuführen, die beinhalten:
Erhalten eines Ausgangsbilds eines Zielobjekts, wobei das Ausgangsbild eine erste Bildqualität aufweist;
Erhalten eines Korrelationsreferenzbilds, das basierend auf einem Referenzbild erzeugt wird, das mit dem Zielobjekt verknüpft ist, wobei das Referenzbild eine zweite Bildqualität aufweist, die höher ist als die erste Bildqualität, und das Korrelationsreferenzbild eine dritte Bildqualität aufweist, die niedriger ist als die zweite Bildqualität;
Bestimmen eines optimierten Bilds des Ausgangsbilds durch Eingeben des Ausgangsbilds und des Korrelationsreferenzbilds in ein Optimierungsmodell, wobei sich das Optimierungsmodell auf ein maschinelles Lernmodell bezieht, das für hochauflösende und rauschreduzierte Rekonstruktion unter Verwendung von im Referenzbild vorhandenen Vorinformationen konfiguriert ist, und das optimierte Bild eine vierte Bildqualität aufweist, die höher ist als die erste Bildqualität, wobei das Optimierungsmodell eine Komponente zur Extraktion tiefer Merkmale und eine Komponente zur Extraktion struktureller Merkmale beinhaltet, wobei die Komponente zur Extraktion tiefer Merkmale konfiguriert ist für:
Extrahieren erster mehrschichtiger Merkmale aus dem Ausgangsbild, um eine erste Merkmalskarte zu erzeugen;
Extrahieren zweiter mehrschichtiger Merkmale aus dem Referenzbild, um eine zweite Merkmalskarte zu erzeugen;
Extrahieren dritter mehrschichtiger Merkmale aus dem Korrelationsreferenzbild, um eine dritte Merkmalskarte zu erzeugen; und
wobei die Komponente zur Extraktion struktureller Merkmale dazu konfiguriert ist, aus dem Ausgangsbild strukturelle Merkmale zu extrahieren, um eine vierte Merkmalskarte zu erzeugen.

14. System (100) nach Anspruch 13, wobei mindestens eines der ersten mehrschichtigen Merkmale, der zweiten mehrschichtigen Merkmale oder dritten mehrschichtigen Merkmale tiefe Merkmale und/oder flache Merkmale beinhaltet.

15. System (100) nach Anspruch 13 oder 14, wobei das Optimierungsmodell eine Komponente zur Korrelationssuche beinhaltet, die dazu konfiguriert ist, eine Korrelationskarte zwischen dem Ausgangsbild und dem Korrelationsreferenzbild basierend auf der ersten Merkmalskarte und der dritten Merkmalskarte zu bestimmen.

## Revendications

1. Procédé d'optimisation d'image, le procédé étant mis en œuvre par un dispositif informatique (200), le procédé comprenant :
l'obtention d'une image initiale d'un objet cible, l'image initiale présentant une première qualité d'image ;
l'obtention d'une image de référence de corrélation qui est générée sur la base d'une image de référence associée à l'objet cible, l'image de référence présentant une deuxième qualité d'image supérieure à la première qualité d'image, et l'image de référence de corrélation présentant une troisième qualité d'image inférieure à la deuxième qualité d'image ;
la détermination d'une image optimisée de l'image initiale en entrant l'image initiale et l'image de référence de corrélation dans un modèle d'optimisation, dans lequel le modèle d'optimisation désigne un modèle d'apprentissage automatique qui est configuré pour une reconstruction haute résolution et à bruit réduit en utilisant des informations a priori existant dans l'image de référence, et l'image optimisée présente une quatrième qualité d'image supérieure à la première qualité d'image, dans lequel le modèle d'optimisation inclut un composant d'extraction de caractéristique profonde et un composant d'extraction de caractéristique structurelle, le composant d'extraction de caractéristique profonde est configuré pour :
extraire de premières caractéristiques multicouches à partir de l'image initiale pour générer une première carte de caractéristique ;
extraire de deuxièmes caractéristiques multicouches à partir de l'image de référence pour générer une deuxième carte de caractéristique ;
extraire des caractéristiques multicouches à partir de l'image de référence de corrélation pour générer une troisième carte de caractéristique ; et
le composant d'extraction de caractéristique structurelle est configuré pour extraire des caractéristiques structurelles à partir de l'image initiale pour générer une quatrième carte de caractéristique.

2. Procédé selon la revendication 1, dans lequel au moins une des premières caractéristiques multicouches, des deuxièmes caractéristiques multicouches ou des troisièmes caractéristiques multicouches inclut des caractéristique profonde et/ou caractéristiques peu profondes.

3. Procédé selon la revendication 1 ou 2, dans lequel le modèle d'optimisation inclut un composant de recherche de corrélation configuré pour déterminer une carte de corrélation entre l'image initiale et l'image de référence de corrélation sur la base de la première carte de caractéristique et de la troisième carte de caractéristique.

4. Procédé selon la revendication 3, dans lequel le modèle d'optimisation inclut en outre un composant de génération d'image configuré pour générer l'image optimisée sur la base de la deuxième carte de caractéristique, de la carte de corrélation et de la quatrième carte de caractéristique.

5. Procédé selon la revendication 4, dans lequel le composant d'extraction de caractéristique profonde est connecté fonctionnellement au composant de génération d'image par l'intermédiaire d'un algorithme d'attention ; et
le procédé comprend en outre :
la génération d'une carte de caractéristique corrélée, en utilisant l'algorithme d'attention, sur la base de la deuxième carte de caractéristique et de la carte de corrélation, dans lequel
une entrée du composant de génération d'image inclut la quatrième carte de caractéristique, la carte de corrélation et la carte de caractéristique corrélée, et
une sortie du composant de génération d'image inclut l'image optimisée.

6. Procédé selon la revendication 5, dans lequel l'algorithme d'attention est configuré pour fusionner des caractéristiques de la deuxième carte de caractéristique et de la carte de corrélation à de multiples échelles.

7. Procédé selon l'une quelconque des revendications 1 à 6, dans lequel le modèle d'optimisation est entraîné en utilisant une pluralité d'échantillons d'entraînement, et
chacun d'au moins un de la pluralité d'échantillons d'entraînement inclut une image d'échantillon d'un objet d'échantillon et une image de référence d'échantillon de l'objet d'échantillon ; de préférence
chacun d'au moins un de la pluralité d'échantillons d'entraînement inclut une image d'échantillon d'un premier objet d'échantillon et une image de référence d'échantillon d'un deuxième objet d'échantillon qui est différent du premier objet d'échantillon.

8. Procédé selon la revendication 1, dans lequel le modèle d'optimisation est généré par :
l'obtention d'une pluralité d'échantillons d'entraînement, chacun incluant une image d'échantillon d'un objet échantillon, une image de référence d'échantillon associée à l'objet échantillon et une image étalon d'échantillon correspondant à l'image d'échantillon, l'image d'échantillon présentant une première qualité d'image, l'image de référence d'échantillon présentant une deuxième qualité d'image supérieure à la première qualité d'image et l'image étalon d'échantillon présentant une troisième qualité d'image supérieure à la première qualité d'image ;
l'obtention d'un modèle d'apprentissage automatique initial ; et
la génération du modèle d'optimisation par apprentissage, en utilisant la pluralité d'échantillons d'apprentissage, le modèle d'apprentissage automatique initial selon un processus d'apprentissage incluant :
pour chacun de la pluralité d'échantillons d'entraînement, la détermination d'une image de référence de corrélation d'échantillon sur la base de l'image de référence d'échantillon, l'image de référence de corrélation d'échantillon présentant une quatrième qualité d'image inférieure à la deuxième qualité d'image ; et
la génération du modèle d'optimisation en utilisant chacun de la pluralité d'échantillons d'entraînement et une image de référence de corrélation d'échantillon correspondante.

9. Procédé selon la revendication 8, dans lequel un composant d'extraction de caractéristique profonde, un composant d'extraction de caractéristique structurelle, un composant de recherche de corrélation et un composant de génération d'image du modèle d'apprentissage automatique initial sont entraînés en parallèle.

10. Procédé selon la revendication 9, dans lequel pour chacun de la pluralité d'échantillons d'entraînement et une image de référence de corrélation d'échantillon correspondante,
le composant d'extraction de caractéristique profonde du modèle d'apprentissage automatique initial est configuré pour
extraire de premières caractéristiques multicouches à partir d'un échantillon d'image de l'échantillon d'entraînement pour générer une première carte de caractéristique d'échantillon ;
extraire de deuxièmes caractéristiques multicouches d'échantillon à partir de l'image de référence d'échantillon de l'échantillon d'entraînement pour générer une deuxième carte de caractéristique d'échantillon ; et
extraire de troisièmes caractéristiques multicouches d'échantillon à partir de l'image de référence de corrélation d'échantillon correspondante pour générer une troisième carte de caractéristique d'échantillon ; de préférence
au moins une parmi les premières caractéristiques multicouches d'échantillon, les deuxièmes caractéristiques multicouches d'échantillon ou les troisièmes caractéristiques multicouches d'échantillon inclut des caractéristiques profondes d'échantillon des caractéristiques peu profondes d'échantillon ;
le composant d'extraction de caractéristique structurelle du modèle d'apprentissage automatique initial est configuré pour extraire des caractéristiques structurelles à partir de l'image d'échantillon de l'échantillon d'entraînement pour générer une quatrième carte de caractéristique d'échantillon ;
le composant de recherche de corrélation du modèle d'apprentissage automatique initial est configuré pour déterminer une carte de corrélation d'échantillon entre l'image d'échantillon de l'échantillon d'entraînement et l'image de référence de corrélation d'échantillon correspondante sur la base de la première carte de caractéristique d'échantillon et de la troisième carte de caractéristique d'échantillon ; et
le composant de génération d'image du modèle d'apprentissage automatique initial est configuré pour générer une image optimisée prédite d'échantillon de l'image d'échantillon de l'échantillon d'entraînement sur la base de la deuxième carte de caractéristique d'échantillon, de la carte de corrélation d'échantillon et de la quatrième carte de caractéristique d'échantillon.

11. Procédé selon l'une quelconque des revendications 8 à 10, dans lequel le processus d'entraînement inclut une pluralité d'itérations, chacune de la pluralité d'itérations incluant :
pour un de la pluralité d'échantillons d'entraînement,
la génération d'une image optimisée prédite d'échantillon en entrant l'échantillon d'entraînement dans un modèle d'apprentissage automatique mis à jour déterminé dans une itération précédente ;
la détermination, sur la base de l'image optimisée prédite d'échantillon et d'une image étalon d'échantillon d'entraînement, d'un résultat d'évaluation d'échantillon du modèle d'apprentissage automatique mis à jour ; et
la mise à jour de valeurs de paramètre du modèle d'apprentissage automatique mis à jour sur la base du résultat d'évaluation d'échantillon.

12. Procédé selon la revendication 11, dans lequel le résultat d'évaluation d'échantillon est déterminé sur la base d'au moins un parmi :
une différence entre l'image optimisée prédite d'échantillon de l'échantillon d'entraînement et l'image étalon d'échantillon de l'échantillon d'entraînement, ou
un temps nécessaire au modèle d'apprentissage automatique mis à jour pour générer l'image optimisée prédite d'échantillon de l'échantillon d'entraînement.

13. Système (100) pour l'optimisation d'image, le système comprenant :
un dispositif de stockage (150) incluant un ensemble d'instructions ;
au moins un processeur (210) en communication avec le dispositif de stockage (150), dans lequel lors de l'exécution de l'ensemble d'instructions, l'au moins un processeur (210) est configuré pour ordonner au système (100) de réaliser des opérations incluant :
l'obtention d'une image initiale d'un objet cible, l'image initiale présentant une première qualité d'image ;
l'obtention d'une image de référence de corrélation qui est générée sur la base d'une image de référence associée à l'objet cible, l'image de référence présentant une deuxième qualité d'image supérieure à la première qualité d'image, et l'image de référence de corrélation présentant une troisième qualité d'image inférieure à la deuxième qualité d'image ;
la détermination d'une image optimisée de l'image initiale en entrant l'image initiale et l'image de référence de corrélation dans un modèle d'optimisation, dans lequel le modèle d'optimisation désigne un modèle d'apprentissage automatique qui est configuré pour une reconstruction haute résolution et à bruit réduit en utilisant des informations a priori existant dans l'image de référence, et l'image optimisée présente une quatrième qualité d'image supérieure à la première qualité d'image, dans lequel le modèle d'optimisation inclut un composant d'extraction de caractéristique profonde et un composant d'extraction de caractéristique structurelle, le composant d'extraction de caractéristique profonde est configuré pour :
extraire de premières caractéristiques multicouches à partir de l'image initiale pour générer une première carte de caractéristique ;
extraire de deuxièmes caractéristiques multicouches à partir de l'image de référence pour générer une deuxième carte de caractéristique ;
extraire des caractéristiques multicouches à partir de l'image de référence de corrélation pour générer une troisième carte de caractéristique ; et
le composant d'extraction de caractéristique structurelle est configuré pour extraire des caractéristiques structurelles à partir de l'image initiale pour générer une quatrième carte de caractéristique.

14. Système (100) selon la revendication 13, dans lequel au moins une des premières caractéristiques multicouches, des deuxièmes caractéristiques multicouches ou des troisièmes caractéristiques multicouches inclut des caractéristique profonde et/ou caractéristiques peu profondes.

15. Système (100) selon la revendication 13 ou 14, dans lequel le modèle d'optimisation inclut un composant de recherche de corrélation configuré pour déterminer une carte de corrélation entre l'image initiale et l'image de référence de corrélation sur la base de la première carte de caractéristique et de la troisième carte de caractéristique.
